(19) Europäisches Patentamt
European Patent Office
Office européen des brevets

(11) EP 4 123 023 A1

(12) **EUROPEAN PATENT APPLICATION**
published in accordance with Art. 153(4) EPC

(43) Date of publication:
**25.01.2023 Bulletin 2023/04**

(21) Application number: **21770458.4**

(22) Date of filing: **12.03.2021**

(51) International Patent Classification (IPC):
***C12N 15/113*** *(2010.01)* ***A61K 31/713*** *(2006.01)*
***A61P 21/04*** *(2006.01)* ***A61P 43/00*** *(2006.01)*
***C12N 15/115*** *(2010.01)*

(52) Cooperative Patent Classification (CPC):
**A61K 31/713; A61P 21/04; A61P 43/00; C12N 15/113; C12N 15/115**

(86) International application number:
**PCT/JP2021/010214**

(87) International publication number:
**WO 2021/187392 (23.09.2021 Gazette 2021/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2020 JP 2020045137**

(71) Applicant: **National University Corporation Tokyo Medical and Dental University**
**Tokyo 113-8510 (JP)**

(72) Inventors:
- **YOKOTA Takanori**
**Tokyo 113-8510 (JP)**
- **NAGATA Tetsuya**
**Tokyo 113-8510 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) HETERONUCLEIC ACID CONTAINING MORPHOLINO NUCLEIC ACID

(57) In one embodiment, an object of the present invention is to provide a double-stranded nucleic acid complex that can have an excellent activity.

In one embodiment, the present invention relates to a double-stranded nucleic acid complex comprising a first nucleic acid strand and a second nucleic acid strand, wherein said first nucleic acid strand is capable of hybridizing to at least part of a target gene or a transcription thereof, has an antisense effect on said target gene or transcription product thereof, and comprises at least two morpholino nucleic acids; and said second nucleic acid strand comprises a base sequence complementary to said first nucleic acid strand; and said first nucleic acid strand is annealed to said second nucleic acid strand.

EP 4 123 023 A1

Fig. 8

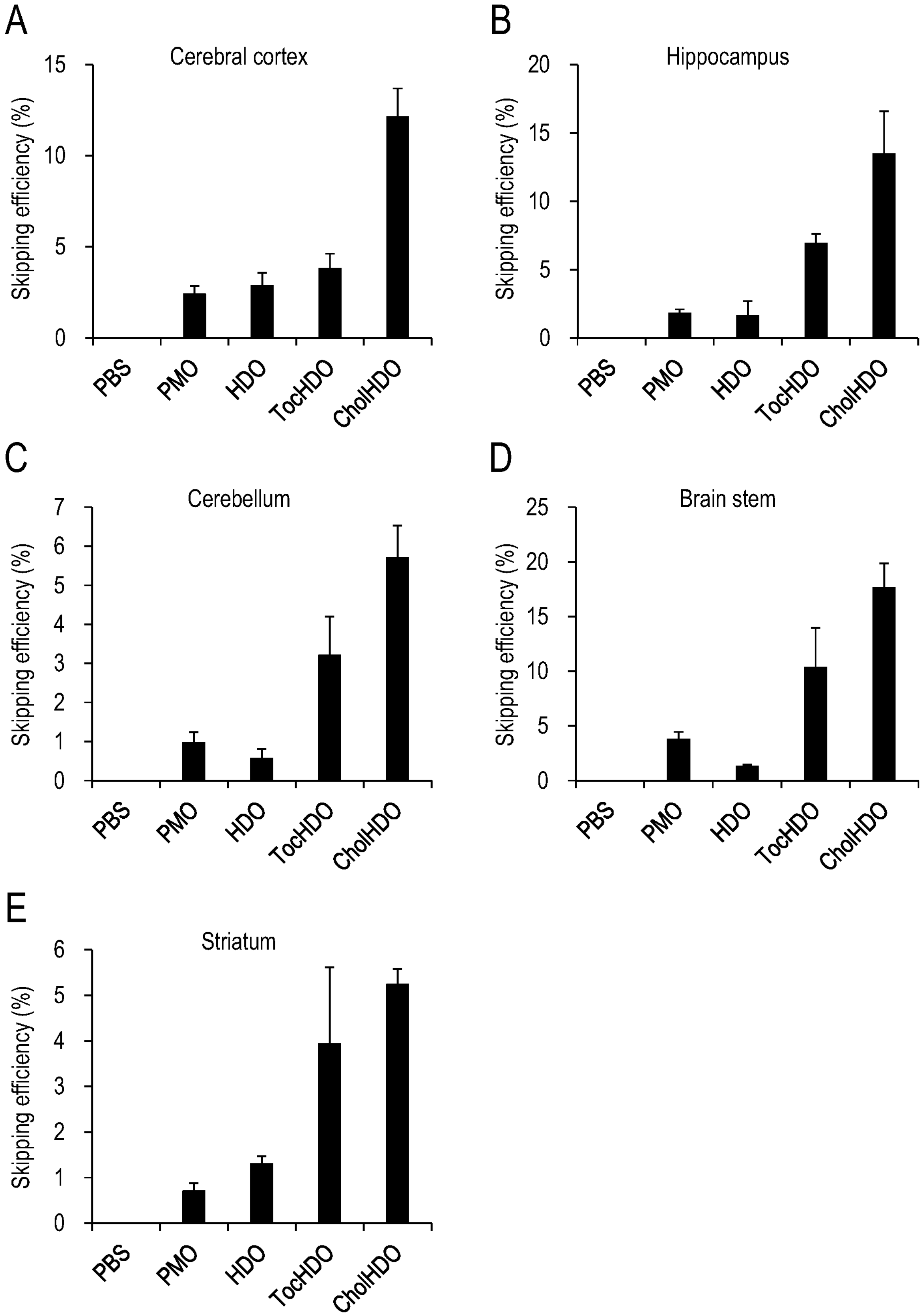
A
Cerebral cortex
Skipping efficiency (%)
0
5
10
15
PBS
PMO
HDO
TocHDO
CholHDO
B
Hippocampus
Skipping efficiency (%)
0
5
10
15
20
PBS
PMO
HDO
TocHDO
CholHDO
C
Cerebellum
Skipping efficiency (%)
0
1
2
3
4
5
6
7
PBS
PMO
HDO
TocHDO
CholHDO
D
Brain stem
Skipping efficiency (%)
0
5
10
15
20
25
PBS
PMO
HDO
TocHDO
CholHDO
E
Striatum
Skipping efficiency (%)
0
1
2
3
4
5
6
PBS
PMO
HDO
TocHDO
CholHDO

## Description

### Technical Field

The present invention relates to a double-stranded nucleic acid complex comprising a morpholino nucleic acid, and a composition or the like comprising the same.

### Background Art

In recent years, an oligonucleotide has been drawing attention in the ongoing development of a pharmaceutical called a nucleic acid medicine, and in particular, development of a nucleic acid medicine utilizing the antisense method is actively carried out from the viewpoint of high selectivity on the target gene and low toxicity. The antisense method is a method comprising selectively modifying or inhibiting the expression of a protein encoded by a target gene or the activity of miRNA by introducing into a cell an oligonucleotide complementary to a target sense strand that is a partial sequence of mRNA or miRNA transcribed from a target gene (antisense oligonucleotide, herein often referred to as "ASO").

As a nucleic acid utilizing the antisense method, the present inventors have developed a double-stranded nucleic acid complex (heteroduplexoligonucleotide, HDO) in which an antisense oligonucleotide and a complementary strand thereto are annealed (Patent Literature 1, Non Patent Literature 1 and 2).

The mechanism of action of the above-described double-stranded nucleic acid complex is not limited, yet partially considered to be as follows. Namely, when a double-stranded nucleic acid complex is introduced into a cell, an RNA region complementary to a portion of an antisense oligonucleotide in the complementary strand is cleaved by RNase H, releasing the antisense oligonucleotide, which can then function to modify the activity or function of a transcription product (see, for example, Patent Literature 2). This is referred to as "RNase H dependent pathway," and for cleavage by RNase H to occur, it is desirable that there are no modifications on the nucleic acid portion. On the other hand, when there are no modifications on the nucleic acid portion, the nucleic acid may be degraded by nucleic acid-degrading enzymes *in vivo* and may not exhibit sufficient activity.

### Citation List

#### Patent Literature

Patent Literature 1: WO2013/089283
Patent Literature 2: WO2014/192310

#### Non-Patent Literature

Non-Patent Literature 1: Nishina K, et. al., "DNA/RNA heteroduplex oligonucleotide for highly efficient gene silencing", Nature Communication, 2015, 6:7969.
Non-Patent Literature 2: Asami Y, et al., "Drug delivery system of therapeutic oligonucleotides", Drug Discoveries & Therapeutics. 2016; 10(5):256-262.

### Summary of Invention

#### Technical Problem

It cannot be said that there is sufficient knowledge about what kind of nucleic acid should be used to suppress degradation, or the like, by nucleic acid-degrading enzymes *in vivo* while maintaining the activity of a double-stranded nucleic acid complex. In one embodiment, an object of the present invention is to provide a novel double-stranded nucleic acid complex which can have an excellent activity.

#### Solution to Problem

The present inventors have found that a double-stranded nucleic acid complex containing morpholino nucleic

acid can have an excellent antisense effect. The present inventors also found that a double-stranded nucleic acid complex comprising an antisense oligonucleotide composed only of morpholino nucleic acids, which does not have an activity due to the RNase H-dependent pathway, unexpectedly has an antisense effect.

The present invention is at least partially based on the above-described findings and provides the following embodiments.

[1] A double-stranded nucleic acid complex comprising a first nucleic acid strand and a second nucleic acid strand, wherein

said first nucleic acid strand is capable of hybridizing to at least part of a target gene or a transcriptional product thereof, has an antisense effect on said target gene or transcriptional product thereof, and comprises at least two morpholino nucleic acids,
said second nucleic acid strand comprises a base sequence complementary to said first nucleic acid strand, and said first nucleic acid strand is annealed to said second nucleic acid strand.

[2] A double-stranded nucleic acid complex comprising a first nucleic acid strand and a second nucleic acid strand, wherein

said first nucleic acid strand is capable of specifically binding to a particular target molecule, has at least one effect of aptamer, decoy, and bait on said target molecule, and comprises at least two morpholino nucleic acids,
said second nucleic acid strand comprises a base sequence complementary to said first nucleic acid strand, and said first nucleic acid strand is annealed to said second nucleic acid strand.

[3] A double-stranded nucleic acid complex according to [1] or [2], not comprising four consecutive natural ribonucleosides.
[4] The double-stranded nucleic acid complex according to any of [1] to [3], wherein 33% or more of the nucleic acids in said first nucleic acid strand are morpholino nucleic acids.
[5] The double-stranded nucleic acid complex according to [4], wherein 100% of the nucleic acids in said first nucleic acid strand are morpholino nucleic acids.
[6] The double-stranded nucleic acid complex according to any of [1] to [5], wherein said second nucleic acid strand is bound to a functional moiety having a function selected from a labeling function, a purification function, and a function of delivery to a target.
[7] The double-stranded nucleic acid complex according to [6], wherein said functional moiety is a lipid.
[8] The double-stranded nucleic acid complex according to [7], wherein said lipid is cholesterol or an analog thereof, or tocopherol or an analog thereof.
[9] The double-stranded nucleic acid complex according to any of [6] to [8], wherein said functional moiety is bound to the 5' end and/or the 3' end of said second nucleic acid strand.
[10] The double-stranded nucleic acid complex according to any of [1] to [9], wherein said first nucleic acid strand and said second nucleic acid strand are bound via a cleavable or uncleavable linker.
[11] The double-stranded nucleic acid complex according to any of [1] to [10], for performing at least one of the following functions in a subject:

suppressing or increasing the expression level of a transcription product or a translation product of the target gene;
inhibiting a function of a transcription product or a translation product of the target gene, regulating RNA splicing; and
inhibiting binding of the target gene to a protein.

[12] The double-stranded nucleic acid complex according to [11], for exon skipping, exon inclusion, steric blocking, and increasing RNA expression.
[13] The double-stranded nucleic acid complex according to any of [1] to [12], for intrathecal administration or intraventricular administration.
[14] A pharmaceutical composition comprising the double-stranded nucleic acid complex according to any of [1] to [13] as an active ingredient.

The contents of the disclosures in Japanese Patent Application No. 2020-045137 which forms the basis for priority of the present application are incorporated herein.

## Advantageous Effects of Invention

The present invention provides a double-stranded nucleic acid complex having a novel structure.

## Brief Description of Drawings

[Figure 1] Figures 1A and 1B are both schematic diagrams showing examples of specific embodiments of a nucleic acid complex of the present invention in which a second nucleic acid strand comprises a lipid.

[Figure 2] Figures 2A to 2C are all schematic diagrams showing examples of specific embodiments of a nucleic acid complex of the present invention in which the second nucleic acid strand contains a lipid and comprises a complementary region and an overhang region.

[Figure 3] Figure 3 is a diagram showing an example of a general mechanism of the antisense method.

[Figure 4] Figure 4 is a diagram showing the structures of various natural nucleotides or non-natural nucleotides.

[Figure 5] Figure 5 is a diagram showing the structures of various bridged nucleic acids.

[Figure 6] Figure 6 shows chemical modifications and structures of oligonucleotides used in Examples 1 and 2.

[Figure 7] Figure 7 shows an exon skipping effect in a cerebrum by systemic administration of heteronucleic acid PMO. PBS refers to a PBS administration group, PMO refers to a single-stranded antisense oligonucleotide (ASO) administration group, Toc HDO refers to a tocopherol-conjugated heteroduplex oligonucleotide administration group, and Chol HDO refers to a cholesterol-conjugated heteroduplex oligonucleotide administration group.

[Figure 8] Figure 8 shows exon-skipping effects of intraventricular administration of heteronucleotic acid PMO in a cerebral cortex (A), a hippocampus (B), a cerebellum (C), a brain stem (D), and a striatum (E). HDO refers to a ligand-free heteroduplex oligonucleotide administration group. The meanings of PBS, PMO, Toc HDO, and Chol HDO are the same as in Figure 7.

[Figure 9] Figure 9 shows antisense effects of intraventricular administration of morpholino nucleic acid-free heteronucleic acid in frontal cortex, occipital cortex, striatum, hippocampus, brain stem, and cerebellum. ASO (mMalat1) refers to an administration group of a ligand-free single-stranded oligonucleotide not comprising a morpholino nucleic acid. PBS refers to a PBS administration group, and Chol HDO (control) refers to an administration group of a cholesterol-conjugated heteroduplex oligonucleotide not comprising a morpholino nucleic acid.

[Figure 10] Figure 10 shows an exon skipping effect of systemic administration of a heteronucleic acid PMO in liver (A) and kidney (B). The meanings of PBS, PMO, Toc HDO, and Chol HDO are the same as in Figure 7.

[Figure 11] Figure 11 shows a chemical modification and the structure of an oligonucleotide used in Example 4.

[Figure 12] Figure 12 shows exon-skipping effects of intraventricular administration of heteronucleic acid PMO in cerebellum (A), brain stem (B), and striatum (C). CholHDO (DNA gap), CholHDO (full OMe), 3'Chol (default), and C3 (default) refer to administration groups of heteroduplex oligonucleotides comprising ASO PMO (mDystrophin) as the first nucleic acid strand and comprising Chol-cRNA (DNA gap), Chol-cRNA (full OMe), 3'Chol-cRNA (default), and C3-cRNA (default) as the second nucleic acid strand, respectively.

[Figure 13] Figure 13 shows exon-skipping effects of intraventricular administration of a heteronucleic acid PMO in hippocampus (A), occipital cortex (B), and cervical spine (C). The meanings of PBS and PMO are the same as in Figure 7. HDO (default), CholHDO (default), CholHDO (DNA gap), CholHDO (full OMe), 3'Chol (default), and C3 (default) refer to administration groups of heteroduplex oligonucleotides comprising ASO PMO (mDystrophin) as the first nucleic acid strand and comprising cRNA (default), Chol-cRNA (default), Chol-cRNA (DNA gap), Chol-cRNA (full OMe), 3'Chol-cRNA (default), and C3-cRNA (default) as the second nucleic acid strand, respectively.

## Description of Embodiments

### 1. Double-stranded nucleic acid complex

#### 1-1. Overview

In one aspect, the present invention relates to a double-stranded nucleic acid complex comprising a first nucleic acid strand and a second nucleic acid strand, wherein said first nucleic acid strand is capable of hybridizing to at least part of a target gene or a transcription product thereof, has an antisense effect on said target gene or transcription product thereof, and comprises at least two morpholino nucleic acids; and said second nucleic acid strand comprises a base sequence complementary to said first nucleic acid strand; and said first nucleic acid strand is annealed to said second nucleic acid strand.

In one aspect, the present invention relates to a double-stranded nucleic acid complex comprising a first nucleic

acid strand and a second nucleic acid strand, wherein said first nucleic acid strand is capable of specifically binding to a particular target molecule, has at least one effect of aptamer, decoy, and bait on said target molecule, and comprises at least two morpholino nucleic acids; and said second nucleic acid strand comprises a base sequence complementary to said first nucleic acid strand; and said first nucleic acid strand is annealed to said second nucleic acid strand.

1-2. Definitions of terms

A "target gene" herein refers to a gene to which the first nucleic acid strand of the double-stranded nucleic acid complex of the present invention can bind. Further, a "target molecule" is a molecule (such as a peptide, a protein, or a nucleic acid) that can be a target of the double-stranded nucleic acid complex of the present invention. A "target gene" or a "target molecule" is a gene or a molecule that can be a target of, for example, an antisense effect or an aptamer, a decoy, or a bait of the double-stranded nucleic acid complex of the present invention.

There is no particular restriction on the kind of a target gene, as long as it is expressed *in vivo.* Examples thereof include a gene which is derived from an organism into which a double-stranded nucleic acid complex of the present invention is to be introduced, such as a gene whose expression is increased in various diseases. Specific examples thereof include a dystrophin gene, a scavenger receptor B1 (often referred to herein as "SR-B1 ") gene, a DMPK (dystrophia myotonic a-protein kinase) gene, and a metastasis associated lung adenocarcinoma transcript 1 (herein often referred to as "Malat1") gene.

Dystrophin gene encode a dystrophin protein, and exon skipping which skips abnormal exons in Duchenne muscular dystrophy is known to be effective in its treatment.

The DMPK gene encodes a myotonin-protein kinase, and is known to be a causative gene for myotonic dystrophy, which is the most frequent type of muscular dystrophy in adults. It is believed that abnormal elongation of the CTG repeat sequence existing in the 3' untranslated region of the DMPK gene is a cause of the disease.

All of the scavenger receptors are receptor membrane proteins for denatured lipoproteins, and are known to be involved in cholesterol and lipoprotein metabolism. SR-B1 is a doublepass transmembrane protein belonging to the evolutionarily conserved CD36 family, and has a long extracellular domain and two short intracellular domains comprising the amino terminal and the carboxyl terminal respectively.

It is known that Malat1 is a long non-coding RNA (lncRNA) which is highly expressed in malignant tumors such as lung cancer, and is localized in the nucleus of myocytes.

A "target transcription product" means herein any RNA that is synthesized by an RNA polymerase and is a direct target of the nucleic acid complex of the present invention. In general, it is a "transcription product of a target gene". Specifically, mRNA transcribed from a target gene (comprising mature mRNA, mRNA precursor, mRNA without base modification, and so on), non-coding RNA (ncRNA) such as miRNA, long non-coding RNA (lncRNA), and natural antisense RNA can be included. Examples of a transcription product of a target gene may comprise pre-mRNA which is a transcription product of the dystrophin gene, DMPK mRNA which is a transcription product of a DMPKgene, SR-B1 mRNA which is a transcription product of the SR-B1 gene, and Malat1 non-coding RNA which is a transcription product of the Malat1 gene.

Specific examples of a target transcription product include the exon 23/intron 23 boundary region of Dystrophin pre-mRNA (GenBank accession number: NC_000086.7), e.g., positions 83803482-83803566, *e.g.*, 83803512-83803536. As other specific examples of a target transcription product, the base sequence of a murine SR-B1 mRNA is shown in SEQ ID NO: 5, and the base sequence of a human SR-B1 mRNA is shown in SEQ ID NO: 6. Further, the base sequence of a murine malat1 non-coding RNA is shown in SEQ ID NO: 7, and a human Malat1 non-coding RNA is shown in SEQ ID NO: 8. Furthermore, the base sequence of a murine DMPK mRNA is shown in SEQ ID NO: 9, and the base sequence of a human DMPK mRNA is shown in SEQ ID NO: 10. In this regard, in all of SEQ ID NOs: 5 to 10, the base sequences of mRNA are replaced with the base sequences of DNA. The base sequence information for these genes and transcription products can be obtained from publicly known databases, such as the database of NCBI (The U.S. National Center for Biotechnology Information).

The base sequence of a publicly known antisense medicine may be also utilized. For example, the base sequence shown in SEQ ID NO: 11 constituting ISIS 598769 (IONIS) which is a therapeutic drug for myotonic dystrophy and related to its causative gene, namely DMPK gene, the base sequence shown in SEQ ID NO: 12 constituting Eteplirsen (Exondys 51, Sarepta Therapeutics), which is known as a therapeutic drug for Duchenne muscular dystrophy and induces exon skipping in pre-mRNA of the dystrophin gene, the base sequence shown in SEQ ID NO: 13 constituting Golodirsen (Sarepta Therapeutics), the base sequence shown in SEQ ID NO: 14 constituting NS-065/NCNP-01 (Nippon Shinyaku Co., Ltd.), or the base sequence shown in SEQ ID NO: 15 constituting Casimersen (Sarepta Therapeutics) may be used.

An "antisense oligonucleotide (ASO)" means herein a single-stranded oligonucleotide that comprises a complementary base sequence capable of hybridizing to all or part, such as any target region, of a target transcriptional product, and can regulate the expression of a transcription product of the target gene or the level of the target transcriptional product by an antisense effect. In the double-stranded nucleic acid complex of the present invention, the first nucleic

acid strand functions as ASO, and its target region may comprise 3'UTR, 5'UTR, exon, intron, coding region, translation initiation region, translation termination region, or any other nucleic acid region. The target region of a target transcriptional product may be at least 8 base in length, for example, 8 to 40 base in length, 10 to 35 base in length, 12 to 25 base in length, 13 to 20 base in length, 14 to 19 base in length, or 15 to 18 base in length.

An "antisense effect" means an effect of regulating expression or editing of a target transcriptional product by hybridization of ASO to the target transcriptional product (*e.g*. RNA sense strand). The phrase "regulating expression or editing of a target transcript" includes suppression or reduction of the expression of a target gene or the expression amount of a target transcription product ("expression amount of a target transcription product" is herein often referred to as "expression level of a target transcription product"), increase, inhibition of translation, functional inhibition of a translation product, regulation of RNA splicing (*e.g*., splicing switching, exon inclusion, and exon skipping), degradation of a transcription product, or inhibition of binding of a target gene to a protein (See Figure 3). For example, in the case of post-transcriptional inhibition of a target gene, when an RNA oligonucleotide is introduced into a cell as ASO, the ASO forms a partial double strand by annealing to mRNA which is a transcription product of a target gene. This partial double strand serves as a cover to prevent translation by ribosomes, so as to inhibit the expression of the target protein encoded by the target gene at the translation level (steric blocking, x mark outside the dashed line in Figure 3). On the other hand, when an oligonucleotide comprising DNA is introduced into a cell as ASO, a partial DNA-RNA heteroduplex is formed. This hetero double-stranded structure is recognized by RNase H, and as a result mRNA of the target gene is degraded and the expression of the protein encoded by the target gene is inhibited at the expression level (within the dashed line in Figure 3). In addition, an antisense effect can also be produced for an intron in an mRNA precursor as a target. Further, an antisense effect can also be produced for miRNA as a target. In this case, as a result of functional inhibition of the miRNA, the expression of the gene whose expression is normally regulated by the miRNA may be increased. In an embodiment, expression regulation of a target transcription product may be decrease of the amount of a target transcription product.

A "translation product of the target gene" means herein any polypeptide or protein that is a direct target of a nucleic acid complex of the present invention and is synthesized by translation of the target transcriptional product or a transcriptional product of the target gene. Examples of a translation product of the target gene comprise a DMPK protein, which is a translation product of the DMPK gene, a SR-B1 protein, which is a translation product of the SR-B1 gene, and a Malat1 protein, which is a translation product of the Malat1 gene.

Herein, "aptamer" refers to a nucleic acid molecule that specifically binds to a particular target molecule intracellular, on the cell membrane, or extracellular, *e.g*., on the cell membrane or extracellular. An aptamer can be produced by a method known in the art, for example, an *in vitro* sorting method using the SELEX (systematic evolution of ligands by exponential enrichment) method.

Herein, "decoy" refers to a nucleic acid having a sequence of binding sites of a transcription factor (*e.g*., NF-kB) or a similar sequence, which is introduced into a cell as a "decoy" to inhibit the function of a transcription factor (inhibit transcription in the case of a transcription activator or promote transcription in the case of a transcription repressor). A decoy nucleic acid can be easily designed based on information about a binding sequence of a target transcription factor.

Herein, "bait" refers to a nucleic acid molecule that specifically binds to a particular target molecule in a cell and modifies a function of the target molecule. A target that interacts with a bait is also referred to as a "prey".

The term "nucleic acid" or "nucleic acid molecule" used herein means a nucleoside or a nucleotide in the case of a monomer, an oligonucleotide in the case of an oligomer, and a polynucleotide in the case of a polymer.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, or uracil as well as other modified nucleobases (modified bases).

A "nucleotide" refers to a molecule in which a phosphate group is covalently bonded to the sugar moiety of the nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Further, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside linkage inside the structure of an oligonucleotide or a polynucleotide.

A "nucleic acid strand" or simply "strand" herein means an oligonucleotide or a polynucleotide. A full length strand or a partial length strand of a nucleic acid strand can be produced by a chemical synthesis using an automated synthesizer, or by an enzymatic step using a polymerase, a ligase, or a restricted reaction. A nucleic acid strand may comprise a natural nucleotide and/or a non-natural nucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "DNA nucleoside" and "RNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase. A nucleic acid strand comprising a non-natural oligonucleotide is in many cases more preferable than a natural type owing to desirable properties, such as enhanced cellular uptake, enhanced affinity for a target nucleic acid, increased stability in the presence of a nuclease, or increase in inhibitory activity.

A "mimic" refers herein to a functional group that substitutes a sugar, a nucleobase, and/or an internucleoside linkage. In general, a mimic is used in place of a sugar or a combination of a sugar-internucleoside linkage, and a nucleobase is maintained for hybridization to a target to be selected. The term "nucleoside mimic" used herein comprises a structure to be used for substituting a sugar at one or more positions of an oligomer compound, or substituting a sugar and a base, or substituting a bond between monomer subunits constituting an oligomer compound. An "oligomer compound" means a polymer composed of linked monomer subunits that can at least hybridize to a region of a nucleic acid molecule. Examples of a nucleoside mimic comprise a morpholino, cyclohexenyl, cyclohexyl, tetrahydropyranyl, bicyclic or tricyclic sugar mimic, such as a nucleoside mimic having a non-furanose sugar unit. Figure 4 shows structures of various natural or non-natural nucleotides.

A "bicyclic nucleoside" herein means a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is herein sometimes referred to as "bridged nucleoside." Some examples of a bridged nucleic acid are shown in Figure 5.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) may be so described that they have a carbon atom at the 2' position and a carbon atom at the 4' position which are bridged with $4'-(CH_2)_p-O-2'$, $4'-(CH_2)_p-CH_2-2'$, $4'-(CH_2)_p-S-2'$, $4'-(CH_2)_p-OCO-2'$, $4'-(CH_2)_n-N(R_3)-O-(CH_2)_m-2'$ [wherein p, m, and n represent integers from 1 to 4, from 0 to 2, and from 1 to 3, respectively; and $R_3$ represents a hydrogen atom, an alkyl group, an alkenyl group, a cycloalkyl group, an aryl group, an aralkyl group, an acyl group, a sulfonyl group, and a unit substituent (*e.g.*, a fluorescently or chemiluminescently labeled molecule, a functional group with nucleic acid cleavage activity, and an intracellular or intranuclear localization signal peptide)]. Furthermore, with respect to a BNA in a certain embodiment, in the $OR_2$ substituent of the carbon atom at the 3' position and the $OR_1$ substituent of the carbon atom at the 5' position, $R_1$ and $R_2$ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or $P(R_4)R_5$ [wherein $R_4$ and $R_5$, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a $C_1$-$C_5$ alkoxy group, a $C_1$-$C_5$ alkylthio group, a $C_1$-$C_6$ cyanoalkoxy group, or an amino group substituted with a $C_1$-$C_5$ alkyl group]. Non-limiting examples of such BNA comprise methyleneoxy($4'-CH_2-O-2'$) BNA (LNA, Locked Nucleic Acid®, also known as 2',4'-BNA), *e.g.*, α-L-methyleneoxy($4'-CH_2O-2'$) BNA or *β-D*-methyleneoxy($4'-CH_2-O-2'$) BNA, ethyleneoxy($4'-(CH2)_2-O-2'$) BNA (also known as ENA), *β-D*-thio($4'-CH_2-S-2'$) BNA, aminooxy($4'-CH_2-O-N(R_3)-2'$) BNA, oxyamino($4'-CH_2-N(R_3)-O-2'$) BNA (also known as 2',4'-$BNA^{NC}$; R=H is 2',4'-$BNA^{NC}$[N-H], R=Me is 2',4'-$BNA^{NC}$[N-Me]), 2',4'-$BNA^{coc}$, 3'-amino-2',4'-BNA, 5'-methyl BNA, ($4'-CH(CH_3)-O-2'$) BNA (also known as cEt BNA), ($4'-CH(CH_2OCH_3)-O-2'$) BNA (also known as cMOE BNA), amido BNA (4'-C(O)-N(R)-2') BNA (R=H, or Me) (also known as AmNA; R=H is AmNA[N-H], R=Me is AmNA[N-Me])), guanidine BNA (also known as GuNA (*e.g.*, R=H is GuNA[N-H], R=Me is GuNA[N-Me]) in Figure 5), amine BNA (also known as 2'-Amino-LNA), 2'-O,4'-C-spirocyclopropylene-bridged nucleic acid (also known as scpBNA), and other BNA known to those skilled in the art. A bicyclic nucleoside having a methyleneoxy($4'-CH_2-O-2'$) bridge is also referred to as LNA nucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside linkage, and a modified nucleobase. The term "nucleotide mimic" herein comprises a structure used to substitute a nucleoside and a bond (linkage) at one or more positions in an

oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity. Therefore, it is more preferable than a natural nucleotide.

A "modified internucleoside linkage" means herein an internucleoside linkage that has a substitution or any change from a naturally occurring internucleoside linkage (*i.e.,* phosphodiester linkage). A modified internucleoside linkage comprises a phosphorus-containing internucleoside linkage that comprises a phosphorus atom, and a phosphorus-free internucleoside linkage that does not comprise a phosphorus atom. Typical phosphorus-containing internucleoside linkages comprise, but not limited to, a phosphodiester linkage, a phosphorothioate linkage, a phosphorodithioate linkage, a phosphotriester linkage, an alkylphosphonate linkage, an alkylthiophosphonate linkage, a boranophosphate linkage, and a phosphorodiamidate. A phosphorothioate linkage is an internucleoside linkage in which an unbridged oxygen atom in a phosphodiester linkage is substituted with a sulfur atom. A method for preparing a phosphorus-containing and phosphorus-free linkage is well known. It is preferable that a modified internucleoside linkage is a linkage having a higher resistance to a nuclease than a naturally occurring internucleoside linkage.

When an internucleoside bond has a chiral center, the internucleoside bond may be chirally controlled. The term "chirally controlled" means that a single diastereomer is present with respect to the chiral center, for example, chirally bound phosphorus. An internucleoside bond chirally controlled may be completely chirally pure, or may have a high chiral purity, for example, a chiral purity of 90% de, 95% de, 98% de, 99% de, 99.5% de, 99.8% de, 99.9% de, or more. A "chiral purity" herein refers to the proportion of one diastereomer in a diastereomer mixture, is represented as the diastereomeric excess rate (% de), and is defined as (diastereomer of interest - Other diastereomers) / (Total diastereomer) $\times$ 100 (%).

An internucleoside bond may be, for example, a phosphorothioate bond chirally controlled to an Rp configuration or Sp configuration. A method for preparing an internucleoside bond chirally controlled is publicly known, and a phosphorothioate bond chirally controlled to an Rp configuration or Sp configuration can be synthesized according to, for example, a method described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, or Yohei Nukaga et al., J. Org. Chem. 2012, 77, 7913-7922. A phosphorothioate bond chirally controlled to an Rp configuration or Sp configuration is also publicly known, and is known to produce an effect described in, for example, Naoki Iwamoto et al., Nat. Biotechnol, . 2017, 35(9), 845-851, or Anastasia Khvorova et al., Nat. Biotechnol., 2017, 35(3), 238-248. For example, in one embodiment, a chirally controlled phosphorothioate linkage in the Sp configuration is more stable than those in the Rp configuration and/or chiral controlled ASOs in the Sp configuration promote target RNA cleavage by RNase H1, resulting in a more sustained response *in vivo.*

A "modified nucleobase" or a "modified base" means herein any nucleobase other than adenine, cytosine, guanine, thymine, or uracil. Examples of a modified nucleobase comprise, but not limited to, 5-methylcytosine, 5-fluorocytosine, 5-bromocytosine, 5-iodocytosine, N4-methylcytosine, N6-methyladenine, 8-bromoadenine, N2-methylguanine, or 8-bromoguanine. A preferred modified nucleobase is 5-methylcytosine.

The term "unmodified nucleobase" or "unmodified base" is synonymous with a natural nucleobase, and means adenine (A) and guanine (G), which are purine bases, and thymine (T), cytosine (C), and uracil (U), which are pyrimidine bases.

A "modified sugar" refers herein to a sugar in which a natural sugar moiety (i.e., sugar moiety found in DNA(2'-H) or RNA(2'-OH)) has undergone a substitution and/or any change. A nucleic acid strand herein may comprise in some cases one or more modified nucleosides comprising a modified sugar. A sugar-modified nucleoside can confer a beneficial biological property such as enhanced nuclease stability, increased binding affinity, or the like to a nucleic acid strand. A nucleoside may comprise a chemically modified ribofuranose ring moiety. Examples of a chemically modified ribofuranose ring comprise, but not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a $C_1$-$C_{12}$ alkyl, or a protecting group), and a combination thereof. Examples of a nucleoside having a modified sugar moiety herein comprise, but not limited to, a nucleoside comprising a substituent such as 5'-vinyl, 5'-methyl (R or S), 4'-S, 2'-F (2'-fluoro group), 2'-$OCH_3$ (2'-OMe group or 2'-O-methyl group), and 2'-$O(CH_2)_2OCH_3$ group. A substituent at the 2' position may be selected from allyl, amino, azide, thio, -O-allyl, -O-$C_1$-$C_{10}$ alkyl, -$OCF_3$, - $O(CH_2)_2SCH_3$, -$O(CH_2)_2$-O-N(Rm)(Rn), and O-$CH_2$-C(=O)-N(Rm)(Rn), wherein Rm and Rn are independently H or a substituted or unsubstituted $C_1$-$C_{10}$ alkyl. A "2'-modified sugar" means herein a furanosyl sugar modified at the 2' position.

In general, a modification can be performed such that nucleotides in the same strand can independently undergo different modifications. In addition, to provide resistance to enzymatic cleavage, the same nucleotide can have a modified internucleoside linkage (*e.g.*, phosphorothioate linkage) and also a modified sugar (*e.g.*, a 2'-O-methyl modified sugar

or a bicyclic sugar). The same nucleotide can also have a modified nucleobase (*e.g.,* 5-methylcytosine) and can also have a modified sugar (*e.g.*, a 2'-O-methyl modified sugar, or a bicyclic sugar).

The number, kind, and position of a non-natural nucleotide in a nucleic acid strand may influence the antisense effect and the like provided by the nucleic acid complex of the present invention. The choice of a modification may vary depending on the sequence of a target gene or the like, but those skilled in the art can determine a suitable embodiment by referring to the descriptions in the literature related to the antisense method (*e.g.*, WO 2007/143315, WO 2008/043753, and WO 2008/049085). Furthermore, when the antisense effect of a nucleic acid complex after the modification is measured and the obtained measured value is not significantly lower than the measured value of the nucleic acid complex before the modification (*e.g.*, when the measured value obtained after the modification is 70% or more, 80% or more, or 90% or more of the measured value of the nucleic acid complex before the modification), a relevant modification may be evaluated.

The term "complementary" as used herein refers to the relationship that nucleobases can form via hydrogen bonds so-called Watson-Crick base pairs (natural base pairs) or non-Watson-Crick base pairs (Hoogsteen base pairs, etc.). The first nucleic acid strand is not necessarily required to be completely complementary to all or part of a target transcriptional product (*e.g.*, the transcription product of a target gene), and it is acceptable if the base sequence has at least 70%, preferably at least 80%, and further preferably at least 90% (*e.g.,* 95%, 96%, 97%, 98%, or 99% or more) in the complementarity. Similarly, the complementary region in the second nucleic acid strand is not necessarily required to be completely complementary to all or part of the first nucleic acid strand, and it is acceptable if the base sequence has a complementarity of at least 70%, preferably at least 80%, and further preferably at least 90% (*e.g.,* 95%, 96%, 97%, 98%, or 99% or more).

"Tocopherol" is herein a methylated derivative of tocorol which is a liposoluble vitamin (vitamin E) having a cyclic structure called chroman. Tocorol has a strong antioxidant effect, and therefore functions *in vivo* as an antioxidant substance to scavenge free radicals produced by metabolism and protect cells from damage.

A plurality of different types of tocopherol are known based on the position of the methyl group bound to chroman comprising $\alpha$-tocopherol, $\beta$-tocopherol, $\gamma$-tocopherol, and $\delta$-tocopherol. A tocopherol herein may be any types of tocopherol. In addition, examples of the analog of tocopherol comprise various unsaturated analogs of tocopherol, such as $\alpha$-tocotrienol, $\beta$-tocotrienol, $\gamma$-tocotrienol, and $\delta$-tocotrienol. Preferably, tocopherol is $\alpha$-tocopherol.

"Cholesterol" is herein a kind of sterol, also called steroid alcohol, which is especially abundant in animals. Cholesterol exerts an important function in the metabolic process *in vivo,* and in animal cells, it is also a major constituent of the membrane system of a cell, together with phospholipid. In addition, the cholesterol analog refers to various cholesterol metabolism products and their analogs, which are alcohols having a sterol backbone. Examples thereof include, but not limited to, cholestanol, lanosterol, cerebrosterol, dehydrocholesterol, and coprostanol.

An "analog" herein refers to a compound having a similar structure and property having the same or a similar basic backbone. The analog comprises, for example, a biosynthetic intermediate, a metabolism product, and a compound having a substituent. Those skilled in the art can determine whether or not a compound is an analog of another compound based on common general technical knowledge.

A "subject" herein refers to the object to which the double-stranded nucleic acid complex or pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Without limitation, a subject may be an individual who needs reduction of the expression level of a target transcription product or regulation of splicing (*e.g.*, exon skipping).

1-3. Configuration

The double-stranded nucleic acid complex of the present invention comprises a first nucleic acid strand and a second nucleic acid strand. The specific configuration of each nucleic acid strand is described below.

In one embodiment, the first nucleic acid strand is a single-stranded oligonucleotide strand that comprises a base sequence capable of hybridizing to a target gene or all or part of the transcription product thereof and produces an antisense effect on the target gene or target transcription product thereof. In another embodiment, the first nucleic acid strand is a single-stranded oligonucleotide strand that can bind specifically to a particular target molecule and has at least one effect of aptamer, decoy, and bait on said target molecule.

The second nucleic acid strand is a single-stranded oligonucleotide strand that comprises a base sequence complementary to the first nucleic acid strand. Also, in a double-stranded nucleic acid complex, the second nucleic acid strand is annealed to the first nucleic acid strand via hydrogen bonds of complementary base pairs. As an example of the present embodiment, there is a heteroduplex oligonucleotide (HDO) disclosed in WO2013/089283, Nishina K, et. al, Nature Communication, 2015, 6:7969, and Asami Y, et al, Drug Discoveries & Therapeutics. 2016; 10(5):256-262 (Figures 1A and B).

In a further embodiment, the second nucleic acid strand can further comprise at least one overhang region located on one or both the 5' end side and the 3' end side of the complementary region. An example of the present embodiment is described in WO2018/062510. The term "overhang region" refers to a region adjacent to a complementary region, namely a nucleotide region in the second nucleotide strand in which the 5' end of the second nucleotide strand extends beyond the 3' end of the first nucleotide strand and/or the 3' end of the second nucleotide strand extends beyond the 5' end of the first nucleotide strand when the first nucleic acid strand and the second nucleic acid strand are annealed to form a double-stranded structure, or protruding from the double-stranded structure. The overhang region in the second nucleic acid strand may be located at the 5' end side of the complementary region (Figure 2A) or at the 3' end side (Figure 2B). The overhang region in the second nucleic acid strand may be located at the 5' end side and 3' end side of the complementary region (Figure 2C).

In one embodiment, the base sequence of the first nucleic acid strand can hybridize (or anneal) to a target transcription product because it is complementary to the base sequence of all or part of the target transcription product. Base sequence complementarity can be determined by using a BLAST program or the like. Those skilled in the art can easily determine the conditions (temperature, salt concentration, and the like) under which two strands can hybridize, taking into account the degree of complementarity between the strands. Furthermore, those skilled in the art can easily design an antisense nucleic acid complementary to a target transcript, for example, based on information about the base sequence of a target gene.

Hybridization conditions may be variously stringent, for example, low-stringent and high-stringent conditions. Low-stringent conditions may be relatively low temperature and high salt concentration conditions, *e.g.*, 30°C, 2 $\times$ SSC, 0.1% SDS. High-stringent conditions may be relatively high temperature and low salt concentration conditions, *e.g.*, 65°C, 0.1 $\times$ SSC, 0.1% SDS. The stringency of hybridization can be adjusted by changing conditions such as temperature and salt concentration. Here, 1 $\times$ SSC contains 150 mM sodium chloride and 15 mM sodium citrate.

There is no particular restriction on the base lengths of the first nucleic acid strand and the second nucleic acid strand, and may be at least 8 base in length, at least 9 base in length, at least 10 base in length, at least 11 base in length, at least 12 base in length, at least 13 base in length, at least 14 base in length, or at least 15 base in length. Further, the base length of the first nucleic acid strand and the second nucleic acid strand may be 40 base in length or less, 35 base in length or less, 30 base in length or less, 25 base in length or less, 24 base in length or less, 23 base in length or less, 22 base in length or less, 21 base in length or less, 20 base in length or less, 19 base in length or less, 18 base in length or less, 17 base in length or less, or 16 base in length or less. The first nucleic acid strand and the second nucleic acid strand may have the same length or different lengths (for example, one of them is shorter or longer by 1 to 3 bases). The double-stranded structure formed by the first nucleic acid strand and the second nucleic acid strand may comprise a bulge. The length can be determined according to the balance between the strength of the antisense effect and the specificity of the nucleic acid strand to the target, among other factors such as cost, and synthesis yield. When a nucleic acid such as an aptamer is bound to the first nucleic acid strand and/or the second nucleic acid strand, the base length of the first nucleic acid strand and the second nucleic acid strand as a whole may be the above-described base length plus the base length of the bound nucleic acid. In this case, the base length of the bound nucleic acid is not limited, and may be, for example, at least 10 base in length, at least 15 base in length, or at least 20 base in length, and may be 100 base in length or less, 80 base in length or less, 60 base in length or less, 40 base in length or less, or 30 base in length or less.

A nucleoside in the first nucleic acid strand and the second nucleic acid strand may be a natural nucleoside (deoxyribonucleoside, ribonucleoside, or both) and/or a non-natural nucleoside.

The internucleoside bond in the first nucleic acid strand and the second nucleic acid strand may be a naturally occurring internucleoside bond and/or a modified internucleoside bond. Without limitation, at least one, at least two, or at least three internucleoside bonds from an end (5' end, 3' end or both the ends) of the first nucleic acid strand and/or the second nucleic acid strand are preferably modified internucleoside bonds. In this regard, for example, two internucleoside bonds from the end of a nucleic acid strand refers to an internucleoside bond closest to the end of the nucleic acid strand, and an internucleoside bond positioned next thereto on the opposite side to the end of the nucleic acid strand. Modified internucleoside bonds in the terminal region of a nucleic acid strand are preferred because they can reduce or inhibit undesired degradation of the nucleic acid strand. In an embodiment, all internucleoside bonds of the first nucleic acid strand and/or the second nucleic acid strand may be modified internucleoside bonds. The modified internucleoside bond may be a phosphorothioate bond.

The first nucleic acid strand and the second nucleic acid strand may comprise, as a whole or in part, a nucleoside mimic or a nucleotide mimic. The nucleotide mimic may be a peptide nucleic acid and/or a morpholino nucleic acid.

The first nucleic acid strand contains at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or at least 25 morpholino nucleic acids. In one embodiment, 25% or more, 33% or more, 40% or more, 50% or more, 60% or more, 70% or more, 80% or more, 90% or more, 95% or more, or 100% of the nucleic acids in the first nucleic acid strand are morpholino nucleic acids. The

internucleoside linkages between morpholino nucleic acids are not limited, and some or all of them may be phosphorodiamidate linkages.

In one embodiment, the first nucleic acid strand does not comprise at least four consecutive nucleosides that are recognized by RNase H when hybridized to a target transcription product. Typically, a region comprising from 4 to 20, from 5 to 16, or from 6 to 12 consecutive nucleosides is recognized by RNase H. Examples of a nucleoside recognized by RNase H include a natural type deoxyribonucleoside. The "at least four consecutive nucleosides" recognized by RNase H can be easily determined by those skilled in the art, for example, by examining whether they are cleaved by RNase H. That a double-stranded nucleic acid agent not comprising "at least four consecutive nucleosides" recognized by RNase H has an antisense effect was unexpected, considering technical common knowledge that an antisense effect of a double-stranded nucleic acid agent is mainly due to an RNase H-dependent pathway.

In one embodiment, natural ribonucleosides of the first nucleic acid strand are less than half of the total length of the first nucleic acid strand, or are not contained.

All nucleosides of the second nucleic acid strand may be composed of ribonucleosides and/or modified nucleosides. All nucleosides of the second nucleic acid strand may be composed of deoxyribonucleosides and/or modified nucleosides and may not contain ribonucleosides. In one embodiment, all nucleosides of the second nucleic acid strand may be composed of deoxyribonucleosides and/or modified nucleosides.

At least one, at least two, at least three, or at least four nucleosides from the end (5' end, 3' end, or both ends) of the second nucleic acid strand may be modified nucleosides. Modified nucleosides may contain modified sugars and/or modified nucleobases. The modified sugar may be a 2'-modified sugar (*e.g.*, a sugar containing a 2'-O-methyl group). The modified nucleobase may also be 5-methylcytosine.

The second nucleic acid strand may be composed of, in order from the 5' end, a modified nucleoside of from 2 to 7 or from 3 to 5 bases in length (*e.g.,* a modified nucleoside containing a 2'-modified sugar), a ribonucleoside or a deoxyribonucleoside (optionally linked by a modified nucleoside linkage) of from 4 to 15 bases in length or from 8 to 12 bases in length, and a modified nucleoside of from 2 to 7 bases in length or from 3 to 5 bases in length (*e.g.,* a modified nucleoside containing a 2'-modified sugar).

At least one (*e.g.,* three) internucleoside linkage from the 3' end of the second nucleic acid strand may be a modified internucleoside linkage such as a phosphorothioate linkage with a high resistance to an RNase. It is preferable that the second nucleic acid strand comprises a modified internucleoside linkage such as a phosphorothioate modification at the 3' end, because the gene inhibition activity of the double-stranded nucleic acid complex is improved.

At least one (*e.g*., three) nucleoside from the 3' end of the second nucleic acid strand may be, for example, a modified nucleoside, such as 2'F-RNA, and 2'-OMe, which have high RNase resistance. It is preferable that a modified nucleoside such as 2'F-RNA, and 2'-OMe is comprised at the 3' end of the second nucleic acid strand, because the gene inhibition activity of the double-stranded nucleic acid complex is improved.

The first nucleic acid strand and the second nucleic acid strand may comprise any combination of the above-described modified internucleoside linkage and modified nucleoside.

The first nucleic acid strand and/or the second nucleic acid strand constituting the double-stranded nucleic acid complex of the present invention may be a mixmer. The term "mixmer" refers to herein a nucleic acid strand that comprises natural nucleosides and non-natural nucleosides with periodically or randomly alternating segment lengths, and does not comprise four or more consecutive deoxyribonucleosides or ribonucleosides. Among mixmers, a mixmer in which the non-natural nucleoside is a bridged nucleoside, and the natural nucleoside is a deoxyribonucleoside is specifically referred to as a "BNA/DNA mixmer". Among mixmers, a mixmer in which the non-natural nucleoside is a peptide nucleic acid and the natural nucleoside is a deoxyribonucleoside is specifically called a "peptide nucleic acid/DNA mixmer". Among mixmers, a mixmer in which the non-natural nucleoside is a morpholino nucleic acid, and the natural nucleoside is a deoxyribonucleoside is specifically referred to as a "morpholino nucleic acid/DNA mixmer". A mixmer is not restricted to comprise only two kinds of nucleosides. A mixmer may comprise any number of kinds of nucleosides irrespective of a natural or modified nucleoside, or a nucleoside mimic. For example, a mixmer may comprise one or two consecutive deoxyribonucleosides separated by a bridged nucleoside (*e.g*., LNA nucleoside). A bridged nucleoside may further comprise a modified nucleobase (*e.g.,* 5-methylcytosine).

The first nucleic acid strand and the second nucleic acid strand may be linked via a cleavable or uncleavable linker. In this case, the first nucleic acid strand and the second nucleic acid strand can be linked via a linker to form a single-strand. However, even in that case, the functional region has the same configuration as the double-stranded nucleic acid complex, and therefore such a single-stranded nucleic acid is herein also encompassed as an embodiment of the double-stranded nucleic acid complex of the present invention. The linker can be any polymer. Examples thereof include a polynucleotide, polypeptide, and alkylene. Specifically, it can be composed of a natural nucleotide such as DNA, and RNA, or a non-natural nucleotide such as a peptide nucleic acid and a morpholino nucleic acid. When a linker consists of a nucleic acid, the chain length of a linker may be at least one base, or a chain length of from 3 to 10 bases or from 4 to 6 bases. It is preferably 4 base in length. In this case, the linker can take the form of a hinge (hairpin loop). The position of the linker can be either on the 5' side or the 3' side of the first nucleic acid strand. For example, in the

case of a configuration in which the second nucleic acid strand is bound to the 5' side of the first nucleic acid strand, the 5' end of the first nucleic acid strand and the 3' end of the second nucleic acid strand are linked via a linker. Further details of the cleavable or uncleavable linker are as described below for functional moieties.

In one embodiment, a functional moiety may be bound to the first nucleic acid strand and/or the second nucleic acid strand, e.g., the second nucleic acid strand. The linkage between the first nucleic acid strand and/or the second nucleic acid strand and the functional moiety may be a direct linkage or an indirect linkage via another substance, and in some embodiments, it is preferable that the first nucleic acid strand and/or the second nucleic acid strand and the functional moiety are directly bound by covalent bonding, ionic bonding, hydrogen bonding, or the like, and from the viewpoint of obtaining a more stable bond, covalent bonding is more preferable.

In some embodiments, the structure of the "functional moiety" is not particularly restricted and imparts a desired function on a double-stranded nucleic acid complex to which it is bound. Examples of the desired function include a labeling function, a purification function, and a delivery function to a target. Examples of a moiety that provides a labeling function include compounds such as a fluorescent protein, and luciferase. Examples of a moiety that provides a purifying function include compounds such as biotin, avidin, His-tag peptide, GST-tag peptide, and FLAG-tag peptide. A molecule having an activity for delivery of a double-stranded nucleic acid complex in an embodiment to a target site is preferably bound as a functional moiety to the first nucleic acid strand and/or the second nucleic acid strand, from the viewpoint that the first nucleic acid strand is efficiently delivered to a target site at a high specificity and expression of a target gene is very effectively suppressed by the nucleic acid. Examples of a moiety for providing a delivery function to a target include lipid, antibody, aptamer, and a ligand to a particular receptor.

In an embodiment, the first nucleic acid strand and/or the second nucleic acid strand, for example, the second nucleic acid strand is bound to a lipid. Examples of a lipid include, but not limited to, tocopherol, cholesterol, fatty acid, phospholipid, and analogs thereof; folic acid, vitamin C, vitamin B1, vitamin B2; estradiol, androstane, and analogs thereof; steroid and an analog thereof; ligand of LDLR, SRBI or LRP1/2; FK-506, and cyclosporine; lipids described in PCT/JP2019/12077 and PCT/JP2019/10392. A lipid may be a tocopherol or an analog thereof and/or a cholesterol or an analog thereof, a substituted or unsubstituted $C_{1-30}$ alkyl group, a substituted or unsubstituted $C_{2-30}$ alkenyl group, or a substituted or unsubstituted $C_{1-30}$ alkoxy group.

A substituted or unsubstituted $C_{1-30}$ alkyl group may be, for example, a $C_{3-15}$, $C_{6-14}$, or $C_{9-13}$ linear alkyl group, where the substituent may be a hydroxy group, a halogen atom, or a $C_{1-3}$ alkyl group.

The second nucleic acid strand bound to a substituted or unsubstituted alkyl group may have a group represented by the following Formula (I).

[Chem. 1]

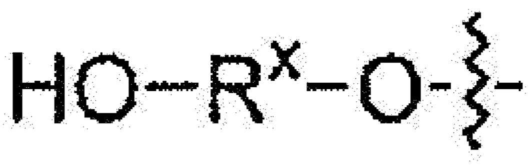

(I)

[wherein $R^x$ is a C3-C24, preferably C6-C14 or C9-C13 linear alkylene group.]

The second nucleic acid strand bound to a substituted or unsubstituted alkyl group may have a group represented by the following Formula (II).

[Chem. 2]

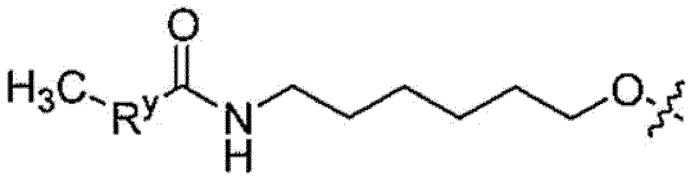

(II)

[wherein $R^y$ is a C1-C15, preferably C3-C15, C6-C14, or C9-C13 linear alkylene group.]

The functional moiety may be linked to the 5' end, or the 3' end, or both ends of the first nucleic acid strand and/or the second nucleic acid strand. Alternatively, the functional moiety may be linked to a nucleotide inside the first

nucleic acid strand and/or the second nucleic acid strand. The first nucleic acid strand and/or the second nucleic acid strand may comprise two or more functional moieties, such as lipids, which may be linked to a plurality of positions in the first nucleic acid strand and/or the second nucleic acid strand, and/or linked as a group to a single position in the first nucleic acid strand and/or the second nucleic acid strand. Functional moieties may be linked to the first nucleic acid strand and/or the second nucleic acid strand, one at the 5' end and one at the 3' end of the first nucleic acid strand.

The linkage between the first nucleic acid strand and/or the second nucleic acid strand and a functional moiety may be a direct linkage or an indirect linkage that is mediated by another substance. However, in a specific embodiment, a functional moiety is preferably directly bound to the first nucleic acid strand and/or the second nucleic acid strand via covalent bonding, ionic bonding, hydrogen bonding, or the like, and from the viewpoint of obtaining a more stable bond, covalent bonding is more preferable.

The functional moiety may also be bound to the first nucleic acid strand and/or the second nucleic acid strand via a cleavable or uncleavable linker. A "cleavable linker" means a linkage group that is cleaved under physiological conditions, *e.g.,* in a cell or in an animal body (*e.g.,* in a human body). In a specific embodiment, a cleavable linker is selectively cleaved by an endogenous enzyme such as a nuclease. Examples of a cleavable linker comprise an amide, an ester, one or both esters of a phosphodiester, a phosphoester, a carbamate, and a disulfide bond, as well as a natural DNA linker.

An "uncleavable linker" refers to a linker that is not cleaved under physiological conditions, for example, in a cell or in an animal body (*e.g.*, in a human body). Examples of an uncleavable linker comprise, but not limited to, a phosphorothioate linkage, modified or unmodified deoxyribonucleosides linked by a phosphorothioate linkage, and a linker consisting of modified or unmodified ribonucleosides. There is no particular restriction on the chain length, when a linker is a nucleic acid such as DNA, or an oligonucleotide, however it may be usually from 2 to 20 base in length, from 3 to 10 base in length, or from 4 to 6 base in length.

Specific examples of the linker comprise a linker represented by the following Formula (I).

[Chem. 3]

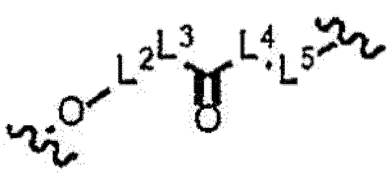

(III)

(where $L^2$ represents a substituted or unsubstituted $C_1$-$C_{12}$ alkylene group (for example, propylene, hexylene, dodecylene), a substituted or unsubstituted $C_3$ -$C_8$ cycloalkylene group (for example, cyclohexylene), $-(CH2)_2-O-(CH_2)_2-O-(CH_2)_2-O-(CH_2)_3-$, $-(CH_2)_2-O-(CH_2)_2-O-(CH_2)_2-O-(CH_2)_2-O-(CH_2)_3-$, or $CH(CH_2-OH)-CH_2-O-(CH_2)_2-O-(CH_2)_2-O-(CH_2)_2-O-(CH_2)_3-$, $L^3$ represents -NH- or a bond, $L^4$ represents a substituted or unsubstituted $C_1$-$C_{12}$ alkylene group (for example, ethylene, pentylene, heptylene, or undecylene), a substituted or unsubstituted $C_3$ -$C_8$ cycloalkylene group (for example, cyclohexylene), $-(CH_2)_2-[O-(CH_2)_2]_m-$, or a bond, wherein m is an integer of 1 to 25, $L^5$ represents -NH-(C=O)-, -(C=O)-, or a bond (here, the substitution is preferably carried out by a halogen atom).

In one embodiment, regarding a linker represented by Formula (III), $L^2$ is unsubstituted $C_3$-$C_6$ alkylene group (for example, propylene, hexylene), $-(CH_2)_2-O-(CH_2)_2-O-(CH_2)_2-O-(CH_2)_3-$, or $-(CH_2)_2-O-(CH_2)_2-O-(CH_2)_2-O-(CH_2)_2-O-(CH_2)_3-$, $L^3$ is -NH-, and $L^4$ and $L^5$ are bonds.

In the double-stranded nucleic acid complex of the present invention, the antisense effect on the target transcript of the first nucleic acid strand can be measured by a method publicly known in the art. For example, after introducing a double-stranded nucleic acid complex into a cell and the like, it can be measured using a publicly known technique such as Northern blotting, quantitative PCR, or Western blotting. By measuring the expression level of a target gene or the level of a target transcriptional product in specific tissues (*e.g.*, the amount of mRNA, the amount of RNA such as microRNA, the amount of cDNA, and the amount of protein), it can be judged whether or not the target gene expression is suppressed by the double-stranded nucleic acid complex in these sites. For example, regarding exon skipping, the effect can be determined by comparing a product produced by exon skipping with a product produced without exon skipping.

An exemplary embodiment of the double-stranded nucleic acid complex of the present invention has been described above, however the double-stranded nucleic acid complex of the present invention is not limited to the above exemplary embodiment.

1-4. Method for producing a double-stranded nucleic acid complex

Those skilled in the art can produce the double-stranded nucleic acid complex of the present invention by appropriately selecting a publicly known method. Usually, without limitation, firstly each of the first nucleic acid strand and the second nucleic acid strand that constitute a double-stranded nucleic acid complex is designed and prepared. For example, the first nucleic acid strand is designed based on the information on the base sequence of the target transcript (*e.g.*, the base sequence of the target gene), and the second nucleic acid strand is designed as a complementary strand thereto. Then, based on the information on the designed base sequences, each nucleic acid strand is synthesized using a commercially available automatic nucleic acid synthesizer, such as that from GE Healthcare, Thermo Fisher Scientific, or Beckman Coulter. Thereafter the prepared oligonucleotides may be purified using a reverse-phase column or the like.

In the case of a double-stranded nucleic acid complex to which a functional moiety is bound, a first nucleic acid strand may be produced according to the above method. Meanwhile, with respect to a second nucleic acid strand to which a functional moiety is bound, it may be produced by performing the aforedescribed synthesis and purification using a nucleic acid species to which a functional moiety has been bound in advance. For example, a second nucleic acid strand may be produced by performing the aforedescribed synthesis and purification using a nucleic acid species to which a functional moiety has been bound in advance. Alternatively, a functional moiety may be joined by a publicly known method to a second nucleic acid strand produced by performing the aforedescribed synthesis and purification. After preparation of each nucleic acid strand, a double-stranded nucleic acid complex to which the functional moiety of interest is bound can be produced by performing annealing described below for the first nucleic acid strand and the second nucleic acid strand.

The method for linking a functional moiety to a nucleic acid is well known in the art. The nucleic acids produced by this method are mixed in an appropriate buffer solution to be denatured at about 90°C to 98°C for several minutes (*e.g.,* 5 min), and then the nucleic acids are annealed in a range of about 30°C to 70°C for about 1 to 8 hours to yield a double-stranded nucleic acid complex of the present invention. Further, a nucleic acid strand can be obtained by ordering from various manufacturers (*e.g.*, GeneDesign Inc.) by specifying the base sequence and the modification site and type. The aforementioned annealing step can be performed by allowing the nucleic acids to stand at room temperature (about 10°C to about 35°C) for about 5 to 60 minutes. It is possible that the first nucleic acid strand and the second nucleic acid strand may be independently dissolved in a buffer solution (*e.g.*, phosphate-buffered saline) or water at about 70°C to 98°C, and the obtained two solutions are mixed, and the mixed liquid is kept at about 70°C to 98°C for several minutes (*e.g.,* 5 minute), and then the same is maintained at about 30°C to 70°C (or 30°C to 50°C) for about 1 to 8 hours to prepare a double-stranded nucleic acid complex of some embodiments of the present invention. It is also possible that the first nucleic acid strand and the second nucleic acid strand are independently dissolved in a buffer solution (*e.g.*, phosphate-buffered saline) or water at room temperature (about 10°C to about 35°C). The annealing conditions (time and temperature) in preparing a double-stranded nucleic acid complex are not limited to the above conditions. In addition, the conditions suitable for promoting annealing of nucleic acid strands are well known in the art.

1-5. Application of double-stranded nucleic acid complex

In one embodiment, the double-stranded nucleic acid complex according to the present invention may be for at least one of the following actions in a subject: suppressing or increasing the expression level of a transcription product or a translation product of a target gene; inhibiting a function of a transcription product or a translation product of a target gene; regulating RNA splicing; and inhibiting binding of a target gene to a protein, such as exon skipping. For example, the double-stranded nucleic acid complex according to the present invention may be for at least one of the following actions: exon skipping, exon inclusion, steric blocking, and increasing RNA expression. The double-stranded nucleic acid complex may be used for providing the above-described action in a particular tissue, *e.g.*, brain, spinal cord, kidney, liver, lung, intestinal tract, spleen, adrenal gland, eye, retina, skin, or peripheral nerves, such as a brain. The brain may be cerebrum, diencephalon, brain stem, or cerebellum, *e.g.*, one or more of the following: cerebrum (*e.g.*, cerebral cortex), brain stem, cerebellum, hippocampus, and striatum. The double-stranded nucleic acid complex of the present invention may be used to provide the above-described action in a tissue other than muscle tissue, including cardiac muscle and skeletal muscle.

In one embodiment, the double-stranded nucleic acid complex of the present invention is for disease or prevention. Examples of a disease include muscular dystrophies (Duchenne muscular dystrophy, myotonic dystrophy type 1 (DM1), Fukuyama muscular dystrophy, facioscapulohumeral muscular dystrophy, limb-girdle muscular dystrophy, or the like), congenital myopathies, primary age-related tauopathies (PART), Alzheimer's disease (AD), progressive supranuclear palsy (PSP), corticobasal degeneration/corticobasal syndrome (CBD), Pick's disease, frontotemporal dementia, neuroinclusion body disease, spinal muscular atrophy (SMA), amyotrophic lateral sclerosis (ALS), Huntington's disease, hereditary spinocerebellar ataxia (SCA), multiple system atrophy, hereditary spastic paraplegia, multiple sclerosis, stroke, epilepsy, and encephalitis.

## 2. Pharmaceutical composition

### 2-1. Overview

In one aspect, the present invention relates to a pharmaceutical composition. The pharmaceutical composition of the present invention comprises the double-stranded nucleic acid complex described herein as an active ingredient. In one embodiment, the pharmaceutical composition described herein may be used for the application of the double-stranded nucleic acid complex described herein. The pharmaceutical composition of the present invention may essentially consist of the double-stranded nucleic acid complex described herein. In other words, the pharmaceutical composition of the present invention may further comprise auxiliary components such as a carrier in addition to the double-stranded nucleic acid complex described herein. The pharmaceutical composition of the present invention may consist solely of the double-stranded nucleic acid complex described herein.

### 2-2. Configuration

The pharmaceutical composition of the present invention can comprise an active ingredient and a carrier. Each component will be described in detail below.

#### 2-2-1. Active ingredient

The pharmaceutical composition of the present invention comprises as an active ingredient at least a double-stranded nucleic acid complex described herein. The pharmaceutical composition of the present invention may comprise two or more kinds of aforedescribed double-stranded nucleic acid complex.

The amount (content) of the aforedescribed double-stranded nucleic acid complex in a pharmaceutical composition varies depending on the kind of double-stranded nucleic acid complex, the site (*e.g.*, brain) to be delivered, the dosage form of the pharmaceutical composition, the dose of the pharmaceutical composition, and the kind of carrier described below. Therefore, it may be determined as appropriate by taking the respective conditions into consideration. Usually, it may be adjusted so that an effective amount of the double-stranded nucleic acid complex is contained in a single dose of the pharmaceutical composition. An "effective amount" may be an amount that is necessary for the double-stranded nucleic acid complex to function as an active ingredient. An "effective amount" may be those having little or no adverse side effects on the living body to which it is applied. This effective amount can vary depending on various conditions such as information on the subject, the administration route, and number of administrations. Ultimately, it may be determined by the judgment of a physician, veterinarian, pharmacist, or the like. "Information on the subject" is various information on an individual of the living body to which the pharmaceutical composition is applied. For example, when the subject is a human, it comprises age, body weight, gender, dietary habit, health status, stage of progression or grade of severity of the disease, drug sensitivity, and presence of a combined drug.

#### 2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH adjuster, a stabilizer, a seasoning, a flavor, an excipient, a vehicle, a preservative, a binder, a diluent, an isotonizing agent, a sedative, a bulking agent, a disintegrating agent, a buffering agent, a coating agent, a lubricant, a colorant, a sweetener, a thickener, a corrective agent, a dissolution aid, and other additives.

The solvent may be any of, for example, water or other pharmaceutically acceptable aqueous solution, and a pharmaceutically acceptable organic solvent. Examples of an aqueous solution comprise a physiological saline, an isotonic solution containing glucose or another additive, a phosphate-buffered saline, and a sodium acetate buffer solution. Examples of the additive comprise D-sorbitol, D-mannose, D-mannitol, sodium chloride, and further a nonionic surfactant at a low concentration, and polyoxyethylene sorbitan fatty acid ester.

The above carrier is used to avoid or decrease degradation of the double-stranded nucleic acid complex, which is an active ingredient, *in vivo* by an enzyme and the like, and additionally to facilitate formulation or administration, and to maintain the dosage form and drug efficacy. Therefore, it may be used as appropriate and as needed.

2-2-3. Dosage form

There is no particular restriction on the dosage form of the pharmaceutical composition of the present invention as long as the double-stranded nucleic acid complex described herein, which is an active ingredient, is not inactivated by degradation or the like, and the pharmacological effect of the active ingredient can be produced *in vivo*.

The specific dosage form varies depending on the administration method and/or medication conditions. The administration methods can be broadly classified into parenteral administration and peroral administration, and the dosage form appropriate for the respective administration methods can be selected.

When the administration method is parenteral administration, the preferred dosage form is liquid formulation which can be administered directly to the target site, or administered systemically via the circulatory system. Examples of the liquid formulation comprise an injectable. An injectable can be formulated by mixing in an appropriate combination with the aforedescribed excipient, elixir, emulsifier, suspending agent, surfactant, stabilizer, pH adjuster, etc. in the form of a unit dose required according to the generally approved pharmaceutical practices. In addition, it may be ointment, plaster, cataplasm, transdermal patch, lotion, inhalant, aerosol, eye drop, and suppository.

When the administration method is oral administration, the preferred dosage form comprises solid preparation (comprising tablet, capsule, drop, and lozenge), granule, dusting powder, powder, and liquid formulation (comprising oral liquid preparation, emulsion formulation, and syrup). In the case of a solid preparation, if necessary, it may take a dosage form with a coating as publicly known in the art, such as a sugar-coated tablet, a gelatin-coated tablet, an enteric-coated tablet, a film-coated tablets, a double layer tablet, and a multilayer tablet.

There is no particular restriction on the specific shape and size of each of the above-mentioned dosage forms, as long as the respective dosage forms are within the ranges of dosage forms publicly known in the art. As for the manufacturing method of the pharmaceutical composition of the present invention, it may be formulated according to the common procedure in the art.

2-3. Dosing form and dose

Herein there is no particular restriction on the preferable dosing form of a pharmaceutical composition. Administration may be systemic or topical. The route of administration may be oral or parenteral. Specific examples of parenteral administration include intravenous, intraarterial, blood transfusion, intraperitoneal, intraventricular, intrathecal, intraocular, intramuscular, subcutaneous (including implantable continuous subcutaneous administration), intradermal, intravesical, transvaginal, rectal, inhalation or intranasal, and tracheal/tracheal administration. When the target site of application of the present invention is the brain, intraventricular or intrathecal administration at the target site is suitable.

When a pharmaceutical composition is applied by administration or ingestion, the administered amount or ingested amount may be, for example, from 0.00001 mg/kg/day to 10000 mg/kg/day, or from 0.001 mg/kg/day to 100 mg/kg/day for the double-stranded nucleic acid complex contained in the pharmaceutical composition. A pharmaceutical composition may be applied by single-dose administration or multiple dose administration. In the case of multiple dose administration, it may be administered daily or at appropriate time intervals (*e.g.*, at intervals of 1 day, 2 days, 3 days, 1 week, 2 weeks, or 1 month), for example, for 2 to 20 times. A single dose of the double-stranded nucleic acid complex described above may be, for example, 0.001 mg/kg or more, 0.005 mg/kg or more, 0.01 mg/kg or more, 0.25 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount in the range of from 0.001 mg/kg to 500 mg/kg (*e.g.,* 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The double-stranded nucleic acid complex of the present invention may be administered twice a week for total four times at a dose of from 0.01 to 10 mg/kg (*e.g.*, about 6.25 mg/kg). Alternatively, the double-stranded nucleic acid complex may be administered once or twice a week for total two to four times, for example at a frequency of twice a week for total two times, at a dose of from 0.05 to 30 mg/kg (*e.g.*, about 25 mg/kg). By adopting such a dosing regimen (divided administration), the toxicity can be lowered (*e.g.*, avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (*e.g.*, half a day or longer)

3. Method

In one aspect, the present invention relates to a method for producing an antisense effect on a target gene or

a transcript thereof, or at least one effect of aptamer, decoy, and bait on a target molecule, comprising administering to a subject a nucleic acid complex or a composition as described herein. The method may be a method for treating or preventing a disease of a subject.

## Examples

In the following, the present invention will be described in more detail by means of Examples. However, the technical scope of the present invention is not limited to these Examples.

### [Example 1: Exon skipping effect by systemic administration of heteronucleic acid PMO in the brain]

In Example 1, an exon skipping effect in the cerebrum was evaluated after multiple doses of a double-stranded nucleic acid complex consisting of an antisense oligonucleotide (phosphorodiamidate morpholino oligomer (hereafter, also referred to as "PMO")) and a tocopherol- or cholesterol-conjugated complementary strand that targets (produces exon-skipping) the exon 23/intron 23 boundary region in an mdx mouse (Duchenne muscular dystrophy model mouse).

(Material and method)

(1) Preparation of nucleic acid agent

A double-stranded nucleic acid agent was compared to a single-stranded antisense oligonucleotide (ASO) control. The control (ASO) was a 25 mer single-stranded morpholino nucleic acid targeting exon 23/intron 23 of the pre-mRNA of the mouse dystrophin gene (dystrophin). This ASO is composed entirely of 25 mer morpholino nucleic acids, and all internucleoside bonds are phosphorodiamidate bonds. This morpholino nucleic acid has a complementary base sequence to positions 83803536-83803512 of mouse Dystrophin pre-mRNA (GenBank accession number: NC_000086.7).

By annealing this morpholino nucleic acid (first nucleic acid strand) with a tocopherol - conjugated Toc-cRNA (mDystrophin) or cholesterol-conjugated Chol-cRNA(mDystrophin), a tocopherol-conjugated heteroduplex oligonucleotide (hereinafter referred to as "Toc-HDO"), or a cholesterol-conjugated heteroduplex oligonucleotide (Chol-HDO), which was a double-stranded nucleic acid agent, was prepared. The first nucleic acid strand was mixed with the second nucleic acid strand in equimolar amounts, the solution was heated at 95°C for 5 min, then cooled to 37°C and maintained for 1 hour allowing the nucleic acid strand to anneal, thereby preparing the above-described double-stranded nucleic acid agent. The annealed nucleic acids were stored at 4°C or on ice. The prepared double-stranded nucleic acid agent is referred to as Toc HDO and Chol HDO.

The sequence, chemical modifications and structure of oligonucleotides used in Example 1 are shown in Table 1 and Figure 6. All the oligonucleotides were manufactured by GeneDesign Inc. (Osaka, Japan).

[Table 1]

| Oligonucleotide name | Sequence (5'-3') | Sequence # |
|---|---|---|
| ASO PMO (mDystrophin) | **g·g·c·c·a·a·a·c·c·t·c·g·g·c·t·t·a·c·c·t·g·a·a·a·t** | 1 |
| Toc-cRNA (mDystrophin) | Toc-A*U*U*UCAGGUAAGCCGAGGUUUG*G*C*C | 2 |
| Chol-cRNA (mDystrophin) | Chol-A*U*U*UCAGGUAAGCCGAGGUUUG*G*C*C | 2 |
| Lower case letter (Bold letter): morpholino nucleic acid •: Phosphorodiamidate bond<br>Upper case letter (Underlined): 2'-O-Me RNA *: phosphorothioate bond Uppercase letter: RNA Toe: Tocopherol Chol: Cholesterol | | |

(2) *In vivo* administration

Mice were 6- to 7-week-old male mdx mice (Clair, Japan) weighing 20 g. All experiments using mice were performed with n=2. Nucleic acid agents were injected intravenously into the mice at a dose of 100 mg/kg each, once a week for a total of 5 times. In addition, mice injected with PBS alone or ASO PMO (mDystrophin) (instead of a double-stranded nucleic acid agent) were also prepared.

(3) RNA expression analysis

Two weeks after the last dose of the nucleic acid agent, a mouse was dissected to isolate the cerebrum. The mRNA was then extracted from each tissue with IsogenII (manufactured by Nippon Gene Co., Ltd.). One-Step RT-PCR was performed on 1 μg of extracted total RNA using the Qiagen One Step RT-PCR Kit (Qiagen). A reaction solution was prepared according to the protocol provided with the kit. LifeECO (Manufactured by Bioer Technology Co. Ltd.) was used as the thermal cycler. The RT-PCR program used was as follows.

42°C for 30 min: Reverse transcription reaction
95 °C for 15 min: Heat denaturation
[94°C for 30 sec, 60°C for 30 sec, 72°C for 60 sec] x 35 cycles: PCR amplification
72°C for 7 min: Final elongation reaction

The base sequences of the forward primer and the reverse primer used for RT-PCR are as follows.

Forward primer: 5'-ATCCAGCAGTCAGAAAGCAAA-3' (SEQ ID NO:3)
Reverse primer: 5'-CAGCCATCCATTTCTGTAAGG-3'(SEQ ID NO:4)

One μl of the reaction product of the above-described RT-PCR was analyzed using the Agilent DNA1000 kit with a Bioanalyzer 2100 (manufactured by Agilent Inc.). The amount of polynucleotide "A" in the band where exon 23 was skipped and the amount of polynucleotide "B" in the band where exon 23 was not skipped were measured. Based on these "A" and "B" measurements, the skipping efficiency was calculated according to the following formula.

$$\text{Skipping efficiency (\%)} = A/(A + B) \times 100$$

(Results)

The results are shown in Figure 7. As shown in Figure 7, exon skipping was observed only in Chol HDO, which is a cholesterol-conjugated heteroduplex oligonucleotide. This indicates that a heteronucleic acid comprising ASO PMO (mDystrophin), which does not transition to the brain on its own, is capable of transitioning to the brain even when administered systemically. The finding of exon skipping activity in Chol HDO, in which all of the first nucleic acid strand is RNaseH-resistant morpholino nucleic acid, was unexpected, considering the common technical knowledge that a major part of an antisense effect of a double-stranded nucleic acid agent is due to an RNase H-dependent pathway.

[Example 2: Exon skipping effect of intraventricular administration of heteronucleic acid PMO in the brain]

In Example 2, an exon skipping effect in the brain was evaluated after a single intraventricular dose of a double-stranded nucleic acid complex consisting of an antisense oligonucleotide (PMO) and a tocopherol- or cholesterol-conjugated complementary strand that targets (produces exon-skipping) the exon 23/intron 23 boundary region in an mdx mouse (Duchenne muscular dystrophy model mouse).

(Material and method)

(1) Preparation of nucleic acid agent

The nucleic acid agent used in the present Example is the same as the nucleic acid agent prepared in Example 1. However, as a comparison, cRNA without a ligand was prepared and annealed with ASO PMO (mDystrophin) in the same manner as in Example 1. The prepared double-stranded nucleic acid agent is referred to as HDO.

The sequence, chemical modifications and structure of oligonucleotides used in Example 2 are shown in Table 2 and Figure 6. All the oligonucleotides were manufactured by Gene Design Inc. (Osaka, Japan).

[Table 2]

| Oligonucleotide name | Sequence (5'-3') | Sequence # |
|---|---|---|
| ASO PMO (mDystrophin) | **g·g·c·c·a·a·a·c·c·t·c·g·g·c·t·t·a·c·c·t·g·a·a·a·t** | 1 |
| Toc-cRNA (mDystrophin) | Toc-A*U*U*UCAGGUAAGCCGAGGUUUG*G*C*C | 2 |

(continued)

| Oligonucleotide name | Sequence (5'-3') | Sequence # |
|---|---|---|
| Chol-cRNA (mDystrophin) | Chol-A*U*U*UCAGGUAAGCCGAGGUUUG*G*C*C | 2 |
| cRNA (mDystrophin) | A*U*U*UCAGGUAAGCCGAGGUUUG*G*C*C | 2 |
| Lower case letter (Bold letter): Morpholino nucleic acid •: Phosphorodiamidate bond<br>Upper case letter (Underlined): 2'-O-Me RNA *: Phosphorothioate bond Uppercase letter: RNA Toe: Tocopherol Chol: Cholesterol | | |

(2) *In vivo* administration

Mice were 6- to 7-week-old male mdx mice (Clair, Japan) weighing 20 g. All experiments using mice were performed with n=3. A nucleic acid agent was injected intraventricularly in the left ventricle of a mouse in a volume of 10 μL (10 nmol as nucleic acid amount). In addition, mice injected with PBS alone, PMO alone, or HDO without a ligand (instead of the double-stranded nucleic acid agent) were also prepared.

(3) RNA expression analysis

Two weeks after the final dose of the nucleic acid agent, a mouse was dissected to isolate the cerebrum, cerebellum, hippocampus, striatum, and brain stem. The mRNA was then extracted from each tissue with IsogenII (manufactured by Nippon Gene Co., Ltd.). One-Step RT-PCR was performed on 1 μg of extracted total RNA using the Qiagen One Step RT-PCR Kit (Qiagen). A reaction solution was prepared according to the protocol provided with the kit. LifeECO (Manufactured by Bioer Technology Co. Ltd.) was used as the thermal cycler. The RT-PCR program used and the forward primer and the reverse primer used for RT-PCR are as described in Example 1.

(Results)

The results are shown in Figure 8. As shown in Figure 8, intraventricular administration of TocHDO and CholHDO had a particularly pronounced exon skipping effect. The increase in the effect of intraventricular administration of PMO by heteronucleic acid formation and ligand addition was unexpected considering the fact that, as shown in Comparative Example 1 below, no increase in the effect of intraventricular administration by heteronucleic acid formation and ligand addition is observed for nucleic acids not containing morpholino nucleic acid.

[Comparative Example 1: Antisense effect of morpholino nucleic acid-free nucleic acid in the brain by intraventricular administration]

In Comparative Example 1, an antisense effect in the brain was evaluated by a single intraventricular administration of a double-stranded nucleic acid complex consisting of an antisense oligonucleotide targeting the malat 1 in C57BL/6 mice and a tocopherol- or cholesterol-conjugated complementary strand.

(Material and method)

(1) Preparation of nucleic acid agent

A 16-mer single-stranded LNA/DNA gapmer (ASO(mMalat1)) (first nucleic acid strand) targeting the malat1 non-coding RNA was prepared. This LNA/DNA gapmer is a 16-base-in-length oligonucleotide containing three LNA nucleosides at the 5' end and three at the 3' end, and 10 DNA nucleosides between them. This LNA/DNA gapmer has a complementary base sequence to positions 1316-1331 of the mouse malat1 non-coding RNA (SEQ ID NO: 1).
A complementary RNA strand (Chol-cRNA(mMalat1)) (second nucleic acid strand) with a sequence complementary to this ASO and with cholesterol covalently bonded at the 5' end was prepared. The second strand is a 16-base-in-length oligonucleotide containing three 2'-O-methyl-modified ribonucleosides at both ends and 10 ribonucleosides between them. The first strand and the second strand were mixed in equimolar amounts, and the solution was heated at 95°C for 5 minutes, then cooled to 37°C and held for 1 hour, thereby annealing the nucleic acid strands and preparing a double-stranded nucleic acid agent. The annealed nucleic acid was stored at room temperature, 4°C or on ice. The prepared double-stranded nucleic acid agent is referred to as Chol HDO (control).
The sequence, chemical modifications and structure of oligonucleotides used in Comparative Example 1 are

shown in Table 3. All the oligonucleotides were manufactured by Gene Design Inc. (Osaka, Japan).

[Table 3]

| Oligonucleotide name | Sequence (5'-3') | Sequence # |
|---|---|---|
| ASO (mMalat1) | 5 (L)*T(L)*A(L)*g*t*t*c*a*c*t*g*a*a*T(L)*G(L)*5(L) | 16 |
| Chol-cRNA (mMalat1) | Chol-G*C*A*UUCAGUGAAC*U*A*G | 17 |
| Uppercase letter (L): LNA (5(L) represents 5-methyl cytosine LNA)<br>Lower case letter: DNA Uppercase letter: RNA<br>Uppercase letter (Underlined): 2'-O-Me RNA *: phosphorothioate bond Chol: cholesterol | | |

(2) *In vivo* administration

Mice were 6- to 7-week-old male C57BL/6 mice weighing 20 g. All experiments using mice were performed with n=4. A nucleic acid agent (25 μg ASO equivalent) was administered intraventricularly to each mouse. In addition, mice injected with PBS alone (instead of the nucleic acid agent) were also prepared.

(3) RNA expression analysis

Seven days after the final dose of the nucleic acid agent, PBS was perfused into a mouse, after which the mouse was dissected to isolate brain regions. The mRNA was then extracted from each tissue according to protocol using a high-throughput fully automated nucleic acid extraction system MagNA Pure 96 (Roche Life Science Co. Ltd.). A cDNA was synthesized using Transcriptor Universal cDNA Master (Roche Life Sciences) in accordance with a protocol. Quantitative RT-PCR was performed by TaqMan (Roche Life Sciences, Inc). Primers used in quantitative RT-PCR were products designed and manufactured by Thermo Fisher Scientific based on various gene numbers. Amplification conditions (temperature and time) were as follows: [95°C for 10 s, 60°C for 30 s, and 72°C for 1 s] × 45 cycles.

Based on the thus obtained quantitative RT-PCR results, the expression levels of mRNA (malat1)/mRNA (ACTB; internal standard gene) were calculated respectively to obtain the relative expression levels. The mean and standard error of the relative expression levels were calculated. Results of the individual groups were compared and the results were further evaluated by t-test.

(Results)

The results are shown in Figure 9. As shown in Figure 9, the previously reported heteroduplex oligonucleotides without morpholino nucleic acid did not show significantly better gene suppression than single-stranded nucleic acid (ASO) not containing morpholino nucleic acid, even when cholesterol was similarly bound to the complementary strand.

[Example 3: Exon skipping effect of systemic administration of heteronucleic acid PMO in liver and kidney]

In Example 3, an exon skipping effect in the liver and kidney was evaluated after multiple doses of a double-stranded nucleic acid complex consisting of an antisense oligonucleotide (PMO) and a tocopherol- or cholesterol-conjugated complementary strand that targets (produces exon-skipping) the exon 23/intron 23 boundary region in an mdx mouse (Duchenne muscular dystrophy model mouse).

Preparation of nucleic acid agents, *in vivo* administration, and RNA expression analysis were performed according to Example 1. However, in the present Example, RNA expression analysis was performed using liver or kidney instead of cerebrum.

The results are shown in Figure 10. As shown in Figure 10, systemic administration of nucleic acid agents had an exon-skipping effect in the liver and kidney, and such an effect was particularly pronounced in TocHDO and CholHDO.

[Example 4: Exon skipping effect of intraventricular administration of heteronucleic acid PMO comprising various complementary strands]

In Example 4, an exon skipping effect was evaluated after intraventricular doses of a double-stranded nucleic acid complex consisting of an antisense oligonucleotide (PMO) and various complementary strands targeting (produces exon-skipping) the exon 23/intron 23 boundary region in an mdx mouse (Duchenne muscular dystrophy model mouse).

The sequence, chemical modifications and structure of oligonucleotides used in Example 4 are shown in Table

4 and Figure 11. All the oligonucleotides were manufactured by Gene Design Inc. (Osaka, Japan).

[Table 4]

| Oligonucleotide name | Sequence (5'-3') | Sequence # |
|---|---|---|
| ASO PMO (mDystrophin) | **g·g·c·c·a·a·a·c·c·t·c·g·g·c·t·t·a·c·c·t·g·a·a·a·t** | 1 |
| cRNA (default) | A*U*U*UCAGGUAAGCCGAGGUUUG*G*C*C | 2 |
| Chol-cRNA (default) | Chol-A*U*U*UCAGGUAAGCCGAGGUUUG*G*C*C | 2 |
| Chol-cRNA (DNA gap) | Chol-A*U*U*ucagguaagccgagguuug*G*C*C | 18 |
| Chol-cRNA (full OMe) | Chol-A*U*U*UCAGGUAAGCCGAGGUUUG*G*C*C | 19 |
| 3'Chol-cRNA (default) | A*U*U*UCAGGUAAGCCGAGGUUUG*G*C*C-Chl | 2 |
| C3-cRNA (default) | C3-A*U*U*UCAGGUAAGCCGAGGUUUG*G*C*C | 2 |
| Lower case letter (Bold letter): Morpholino nucleic acid •: Phosphorodiamidate bond<br>Uppercase letter (Underlined): 2'-O-Me RNA *: Phosphorothioate bond<br>Lower case letter: DNA Uppercase letter: RNA Toe: Tocopherol Cho!: Cholesterol | | |

The 3' end structure of the oligonucleotide 3'Chol-cRNA (default) shown in Table 4 is shown in Formula (IV) below.

[Chem. 4]

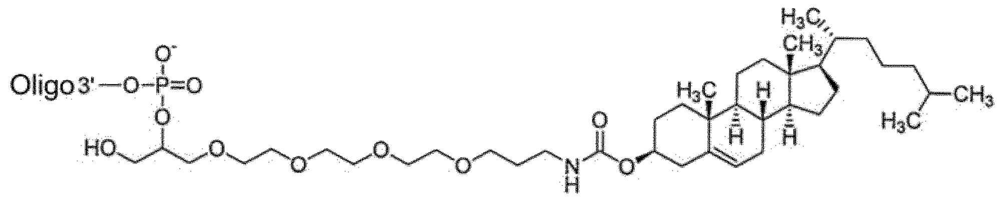
(IV)

The 5' end structure of the oligonucleotide C3-cRNA (default) shown in Table 4 is shown in Formula (V) below.

[Chem. 5]

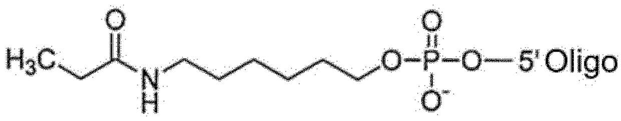
(V)

A heteroduplex oligonucleotide comprising ASO PMO (mDystrophin) as the first nucleic acid strand and various complementary strands shown in Table 4 as the second nucleic acid strand was prepared. A nucleic acid agent was administered intraventricularly in a volume of 10 μL (10 nmol as nucleic acid amount) into the left lateral ventricle of a mouse. In addition, a mouse injected with PBS alone (instead of the double-stranded nucleic acid agent) or PMO alone was also prepared. One week after the nucleic acid agent administration, the mice were dissected to isolate the cerebellum, brain stem, striatum, hippocampus, occipital cortex, and cervical spine for RNA expression analysis. Experiments using mice were performed with n = 2 to 3. Other methods of nucleic acid agent preparation, *in vivo* administration, and RNA expression analysis were in accordance with Example 1.

The results are shown in Figures 12 to 13. In Figures 12 to 13, heteroduplex oligonucleotides containing ASO PMO (mDystrophin) shown in Table 4 as the first nucleic acid strand and cRNA (default), Chol-cRNA (default), Chol-cRNA (DNA gap), Chol-cRNA (full OMe), 3'Chol-cRNA (default), and C3-cRNA (default) shown in Table 4 as the second nucleic acid strand are shown as HDO (default), CholHDO (default), CholHDO (DNA gap), CholHDO (full OMe), 3'Chol (default), and C3 (default), respectively.

As shown in Figures 12 to 13, CholHDO (DNA gap), CholHDO (full OMe), 3'Chol (default), and C3 (default)

had an exon skipping effect in the cerebellum, brain stem, striatum, hippocampus, occipital cortex, and cervical spine, and such an effect was particularly pronounced in CholHDO (DNA gap).

All publications, patents, and patent applications cited herein are incorporated herein directly by reference.

## Claims

1. A double-stranded nucleic acid complex comprising a first nucleic acid strand and a second nucleic acid strand, wherein

   said first nucleic acid strand is capable of hybridizing to at least part of a target gene or a transcription product thereof, has an antisense effect on said target gene or transcription product thereof, and comprises at least two morpholino nucleic acids,
   said second nucleic acid strand comprises a base sequence complementary to said first nucleic acid strand, and
   said first nucleic acid strand is annealed to said second nucleic acid strand.

2. A double-stranded nucleic acid complex comprising a first nucleic acid strand and a second nucleic acid strand, wherein

   said first nucleic acid strand is capable of specifically binding to a particular target molecule, has at least one effect of aptamer, decoy, and bait on said target molecule, and comprises at least two morpholino nucleic acids,
   said second nucleic acid strand comprises a base sequence complementary to said first nucleic acid strand, and
   said first nucleic acid strand is annealed to said second nucleic acid strand.

3. The double-stranded nucleic acid complex according to claim 1 or 2, wherein said double-stranded nucleic acid complex does not comprise four consecutive natural ribonucleosides.

4. The double-stranded nucleic acid complex according to any one of claims 1 to 3, wherein 33% or more of the nucleic acids in said first nucleic acid strand are morpholino nucleic acids.

5. The double-stranded nucleic acid complex according to claim 4, wherein 100% of the nucleic acids in said first nucleic acid strand are morpholino nucleic acids.

6. The double-stranded nucleic acid complex according to any one of claims 1 to 5, wherein said second nucleic acid strand is bound to a functional moiety having a function selected from a labeling function, a purification function, and a function of delivery to a target.

7. The double-stranded nucleic acid complex according to claim 6, wherein said functional moiety is a lipid.

8. The double-stranded nucleic acid complex according to claim 7, wherein said lipid is cholesterol or an analog thereof, or tocopherol or an analog thereof.

9. The double-stranded nucleic acid complex according to any one of claims 6 to 8, wherein said functional moiety is bound to the 5’ end and/or the 3’ end of said second nucleic acid strand.

10. The double-stranded nucleic acid complex according to any one of claims 1 to 9, wherein said first nucleic acid strand and said second nucleic acid strand are bound via a cleavable or uncleavable linker.

11. The double-stranded nucleic acid complex according to any one of claims 1 to 10, for performing at least one of the following functions in a subject:

    suppressing or increasing the expression level of a transcription product or a translation product of the target gene,
    inhibiting a function of a transcription product or a translation product of the target gene,
    regulating RNA splicing, and
    inhibiting binding of the target gene to a protein.

12. The double-stranded nucleic acid complex according to claim 11, for exon skipping, exon inclusion, steric blocking,

and increasing RNA expression.

13. The double-stranded nucleic acid complex according to any one of claims 1 to 12, for intrathecal administration or intraventricular administration.

14. A pharmaceutical composition comprising the double-stranded nucleic acid complex according to any one of claims 1 to 13 as an active ingredient.

Fig. 1

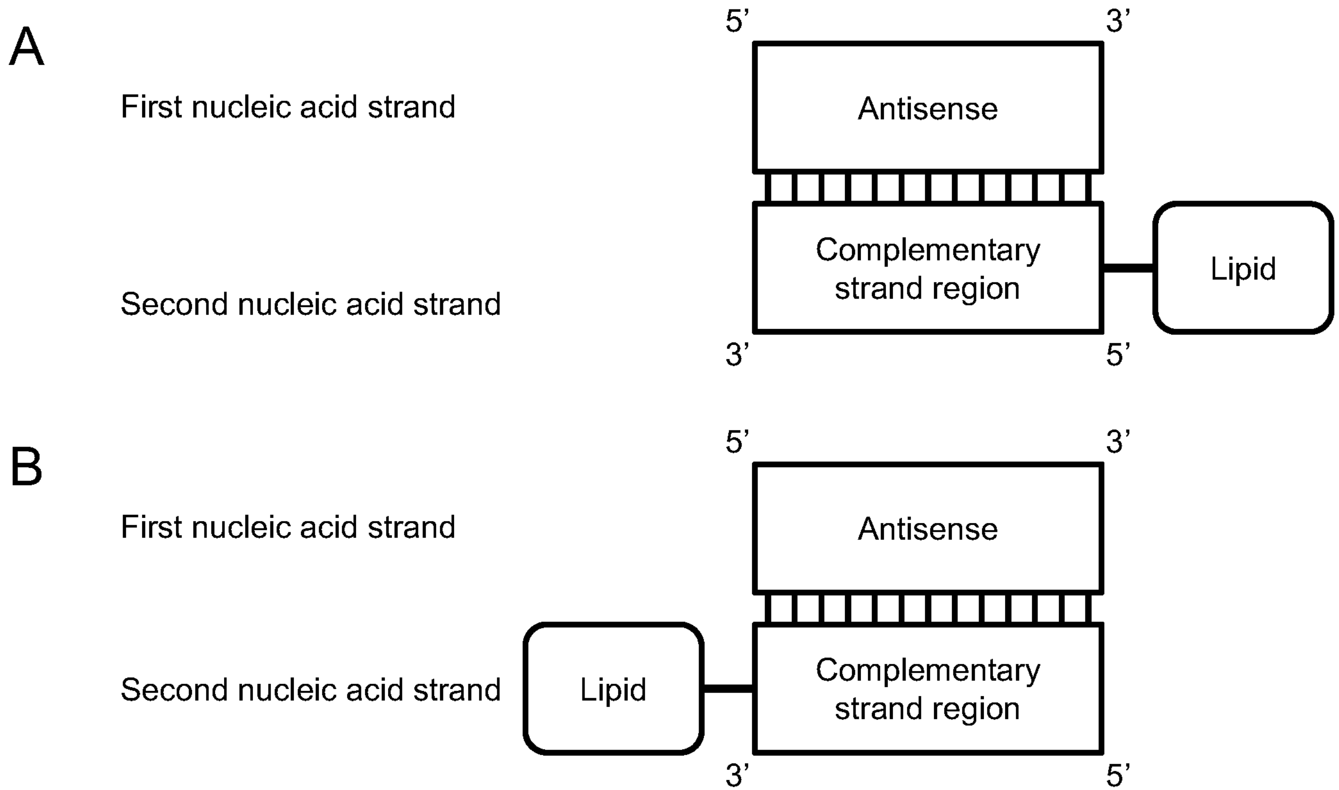
A
First nucleic acid strand
Second nucleic acid strand
5'
3'
Antisense
Complementary strand region
Lipid
3'
5'
B
First nucleic acid strand
Second nucleic acid strand
5'
3'
Antisense
Complementary strand region
Lipid
3'
5'

Fig. 2

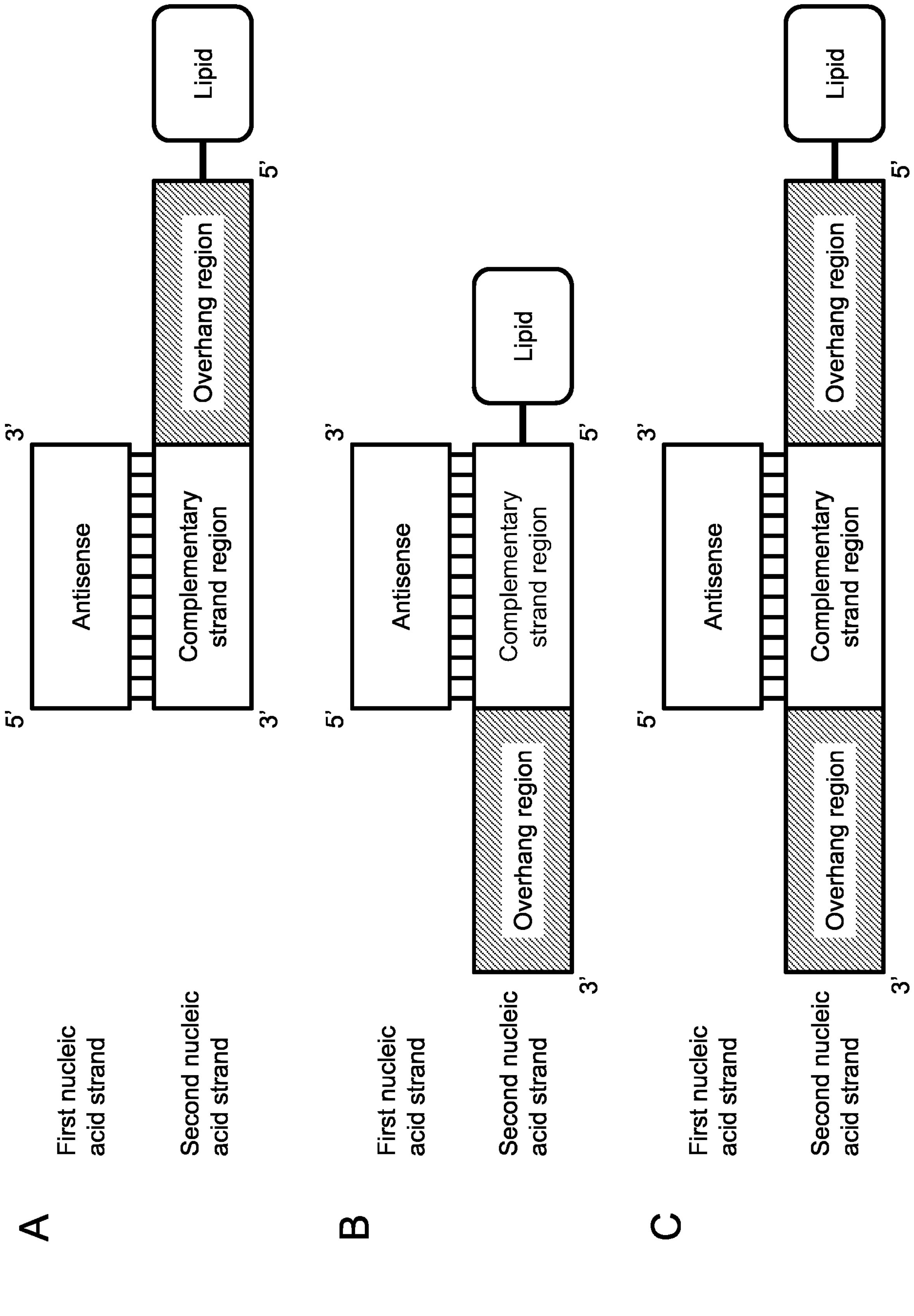
A
First nucleic acid strand
Second nucleic acid strand
5'
3'
Antisense
Complementary strand region
3'
Overhang region
5'
Lipid
B
First nucleic acid strand
Second nucleic acid strand
5'
3'
Antisense
3'
Overhang region
Complementary strand region
5'
Lipid
C
First nucleic acid strand
Second nucleic acid strand
5'
3'
Antisense
3'
Overhang region
Complementary strand region
Overhang region
5'
Lipid

Fig. 3

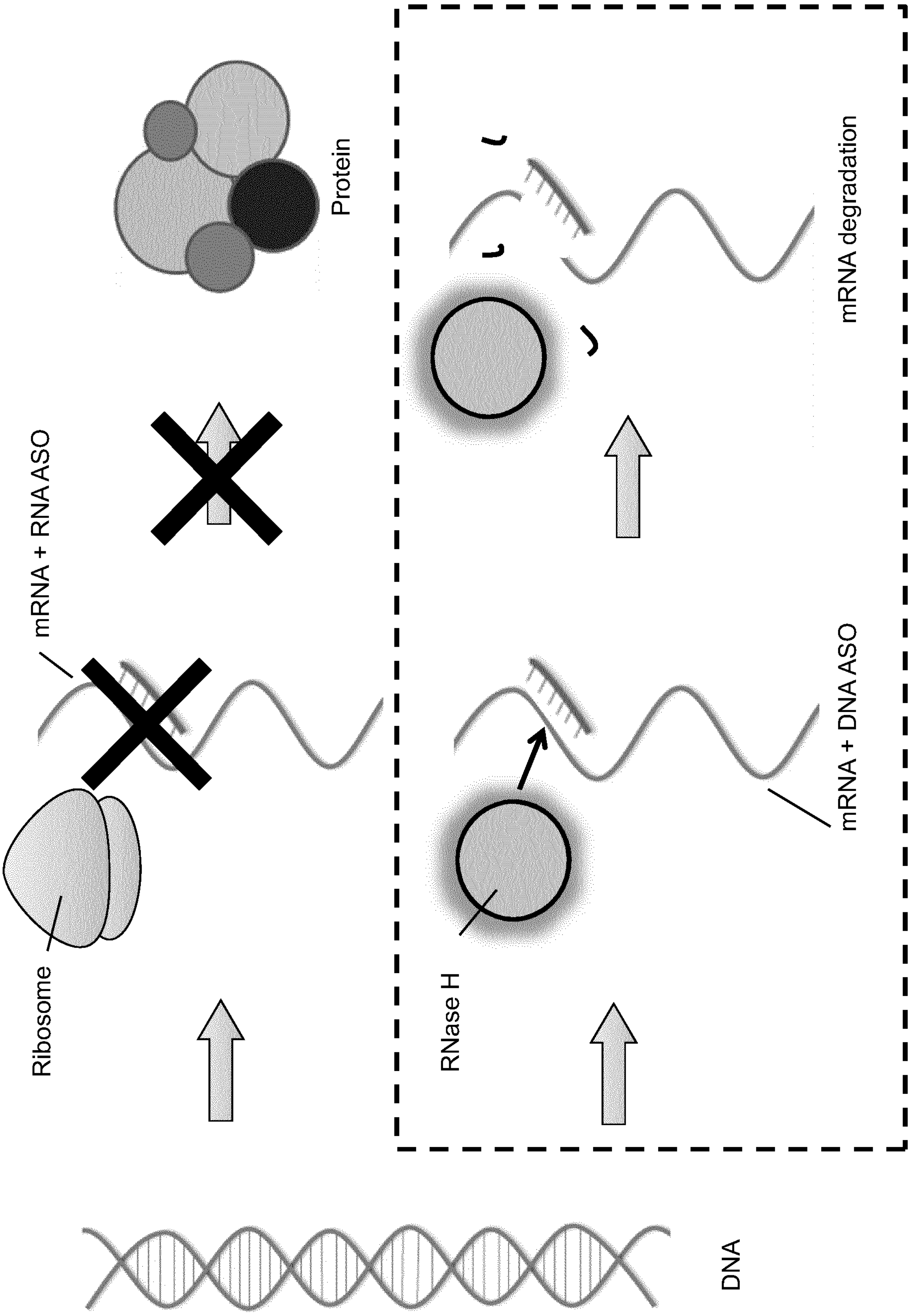
Protein
mRNA + RNA ASO
Ribosome
RNase H
mRNA + DNA ASO
mRNA degradation
DNA

Fig. 4

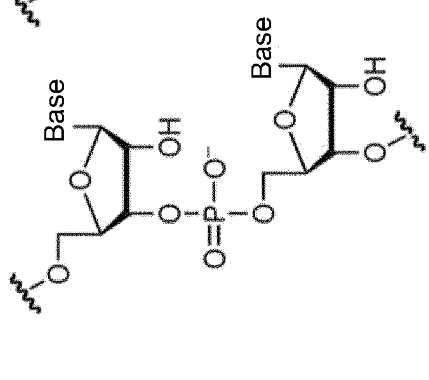
Phosphodiester

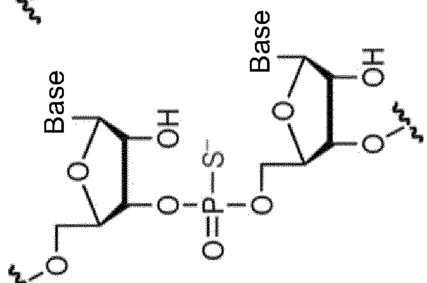
Phosphorothioate

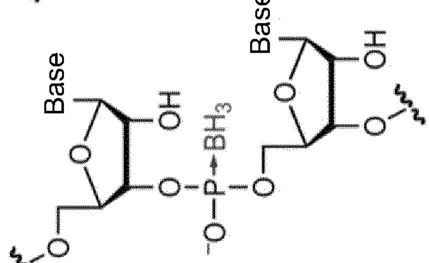
Boranophosphate

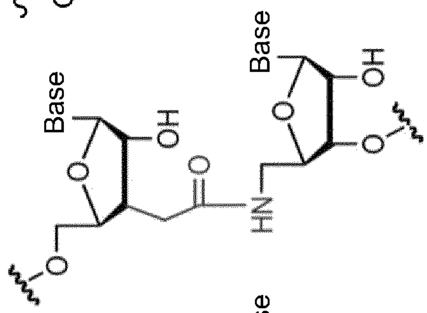
Amide bond

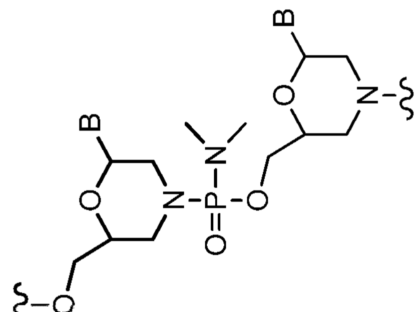
Morpholino

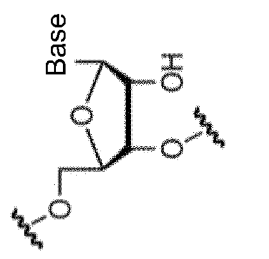
RNA

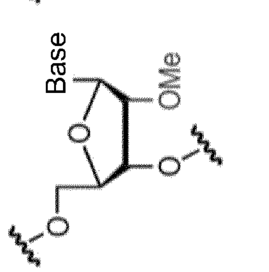
2'-O-Me-RNA

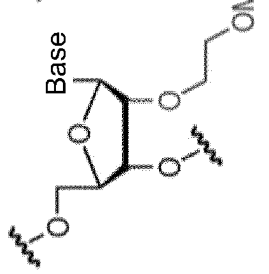
2'-O-MOE-RNA

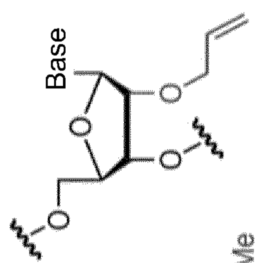
2'-O-allyl-RNA

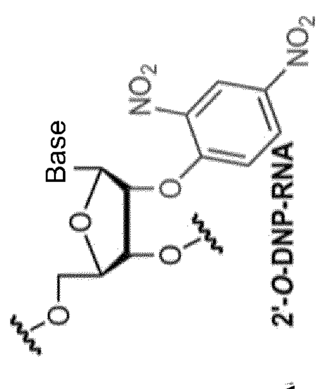
2'-O-DNP-RNA

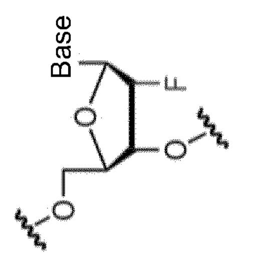
2'F-RNA

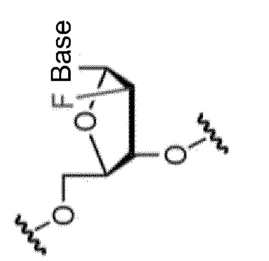
2'F-ANA

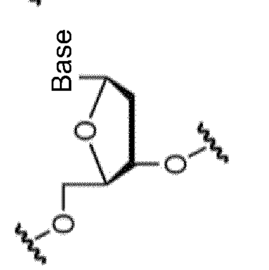
DNA

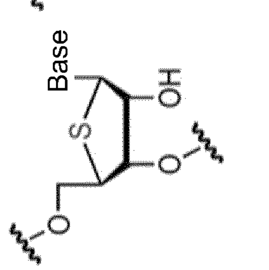
4'S-RNA

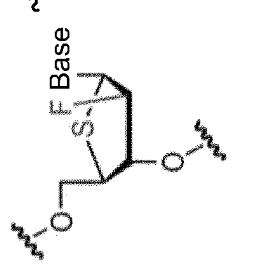
4'S-FANA

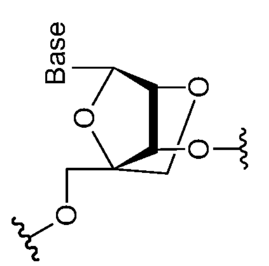
LNA

# Fig. 5

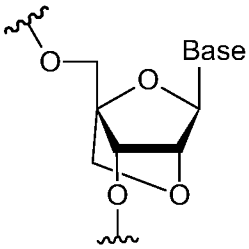

LNA

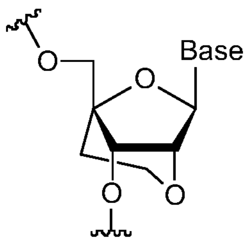

ENA

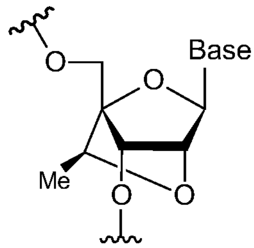

cEt

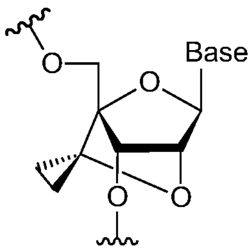

2'-O,4'-C-
spirocyclopropylene
bridged nucleic acid
(scpBNA)

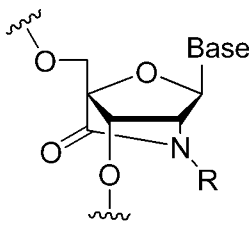

Amide bridged
nucleic acid
(AmNA)
(R=H is AmNA [N-H],
R=Me is AmNA [N-Me])

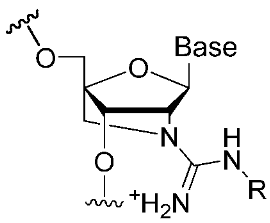

Guanidine bridged
nucleic acid
(GuNA)
(R=H is GuNA [N-H],
R=Me is GuNA [N-Me])

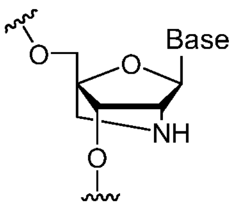

Amine bridged
nucleic acid
(2′- Amino-LNA)

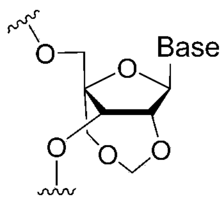

2',4'- BNA$^{COC}$

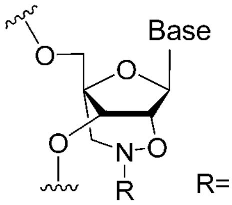

R=Me, H

2',4'- BNA$^{NC}$
(R=H is 2',4'-BNA$^{NC}$ [N-H],
R=Me is 2',4'-BNA$^{NC}$ [N-Me])

Fig. 6

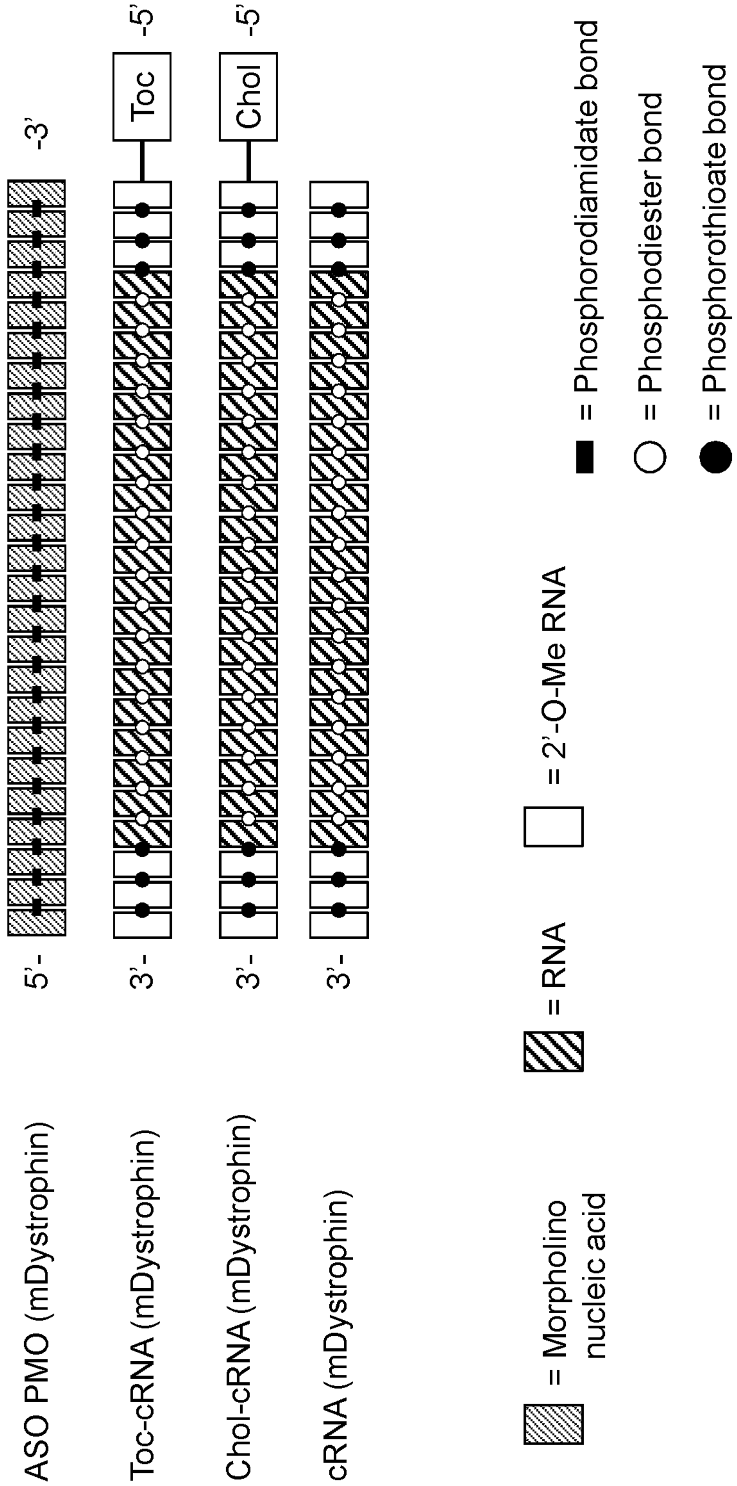
ASO PMO (mDystrophin)
Toc-cRNA (mDystrophin)
Chol-cRNA (mDystrophin)
cRNA (mDystrophin)
5'-
-3'
3'-
-5'
Toc
Chol
= Morpholino nucleic acid
= RNA
= 2'-O-Me RNA
= Phosphorodiamidate bond
= Phosphodiester bond
= Phosphorothioate bond

Fig. 7

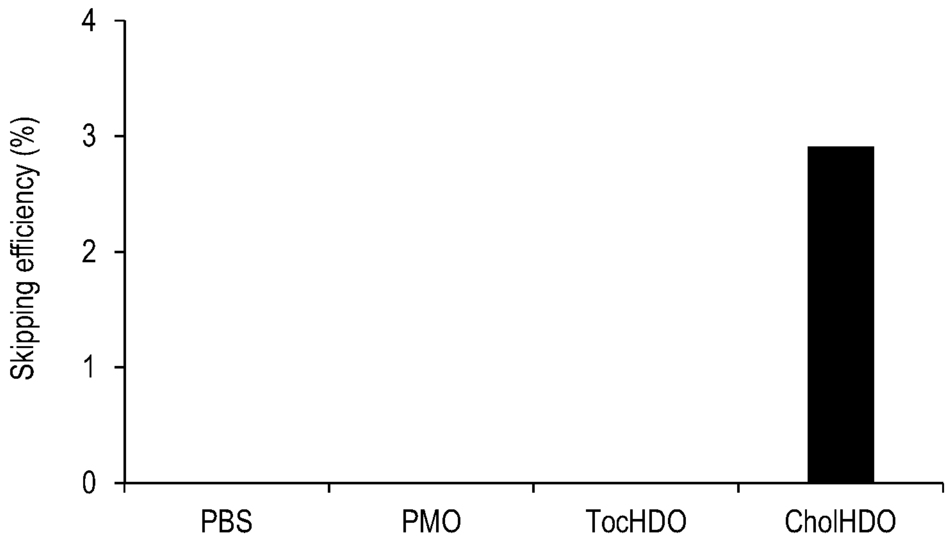

Fig. 8

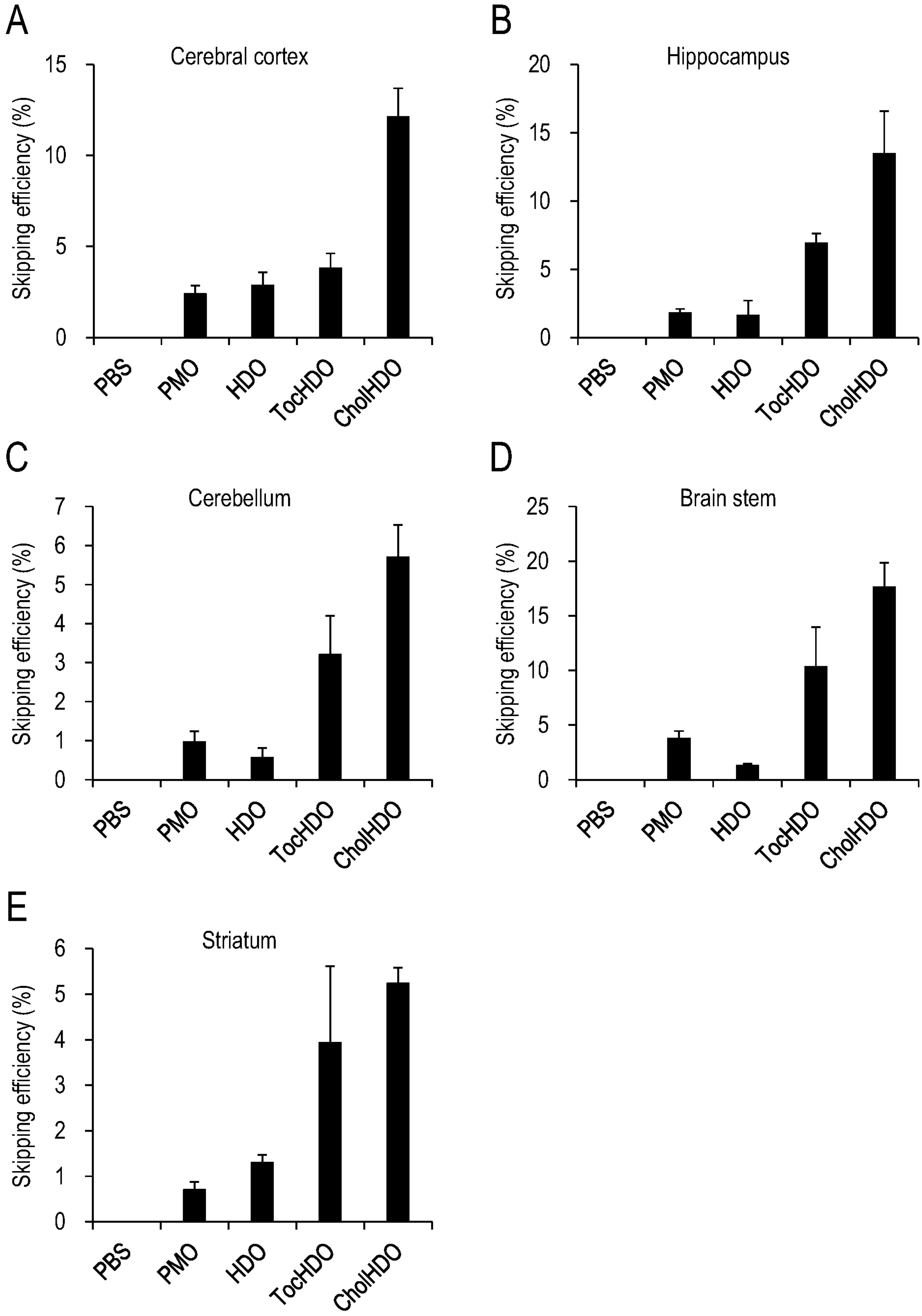
A
Cerebral cortex
Skipping efficiency (%)
15
10
5
0
PBS
PMO
HDO
TocHDO
CholHDO
B
Hippocampus
Skipping efficiency (%)
20
15
10
5
0
PBS
PMO
HDO
TocHDO
CholHDO
C
Cerebellum
Skipping efficiency (%)
7
6
5
4
3
2
1
0
PBS
PMO
HDO
TocHDO
CholHDO
D
Brain stem
Skipping efficiency (%)
25
20
15
10
5
0
PBS
PMO
HDO
TocHDO
CholHDO
E
Striatum
Skipping efficiency (%)
6
5
4
3
2
1
0
PBS
PMO
HDO
TocHDO
CholHDO

Fig. 9

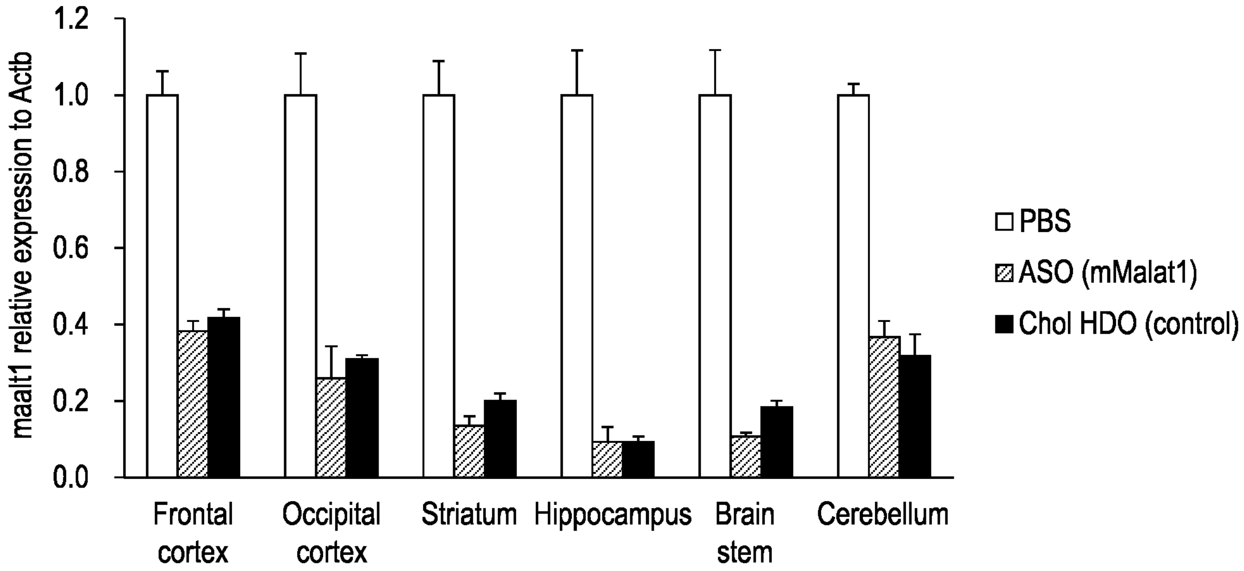
1.2
1.0
0.8
0.6
0.4
0.2
0.0
maalt1 relative expression to Actb
Frontal cortex
Occipital cortex
Striatum
Hippocampus
Brain stem
Cerebellum
PBS
ASO (mMalat1)
Chol HDO (control)

Fig. 10

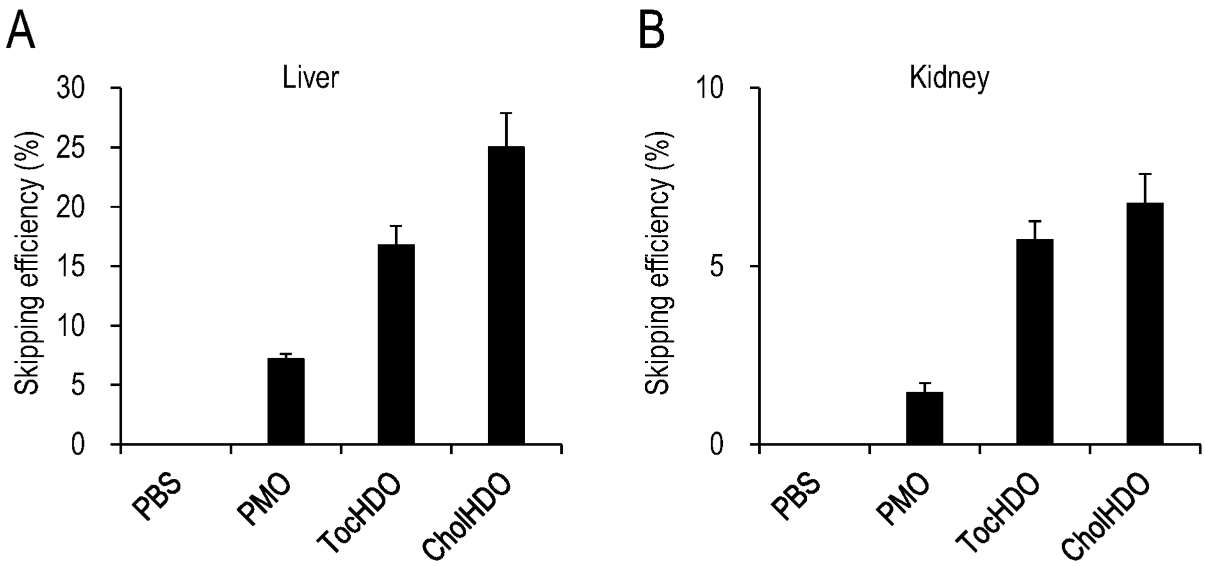
A
Liver
Skipping efficiency (%)
30
25
20
15
10
5
0
PBS
PMO
TocHDO
CholHDO
B
Kidney
Skipping efficiency (%)
10
5
0
PBS
PMO
TocHDO
CholHDO

Fig. 11

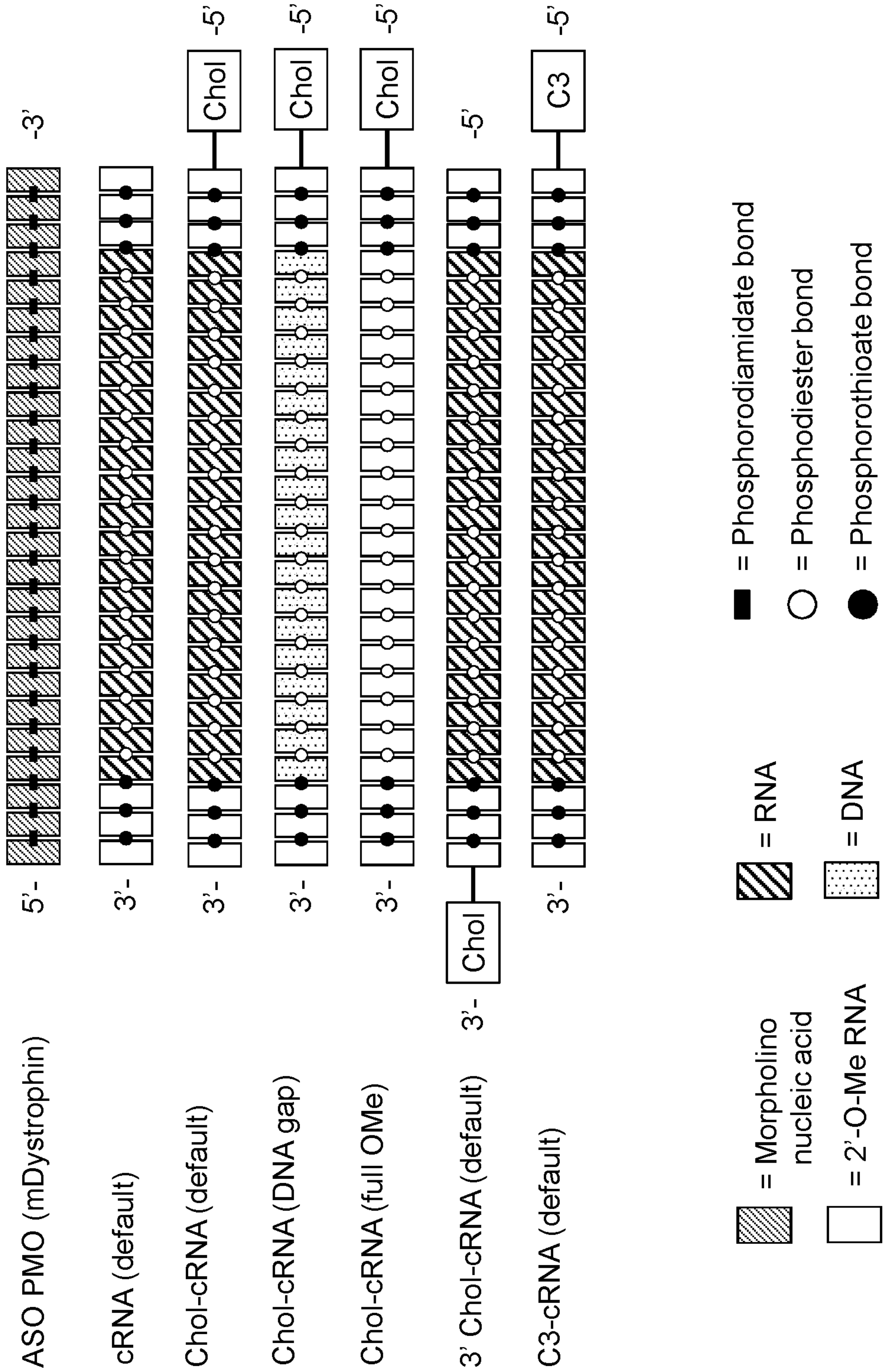
ASO PMO (mDystrophin)
cRNA (default)
Chol-cRNA (default)
Chol-cRNA (DNA gap)
Chol-cRNA (full OMe)
3' Chol-cRNA (default)
C3-cRNA (default)
5'-
-3'
3'-
-5'
Chol
C3
= Morpholino nucleic acid
= 2'-O-Me RNA
= RNA
= DNA
= Phosphorodiamidate bond
= Phosphodiester bond
= Phosphorothioate bond

Fig. 12

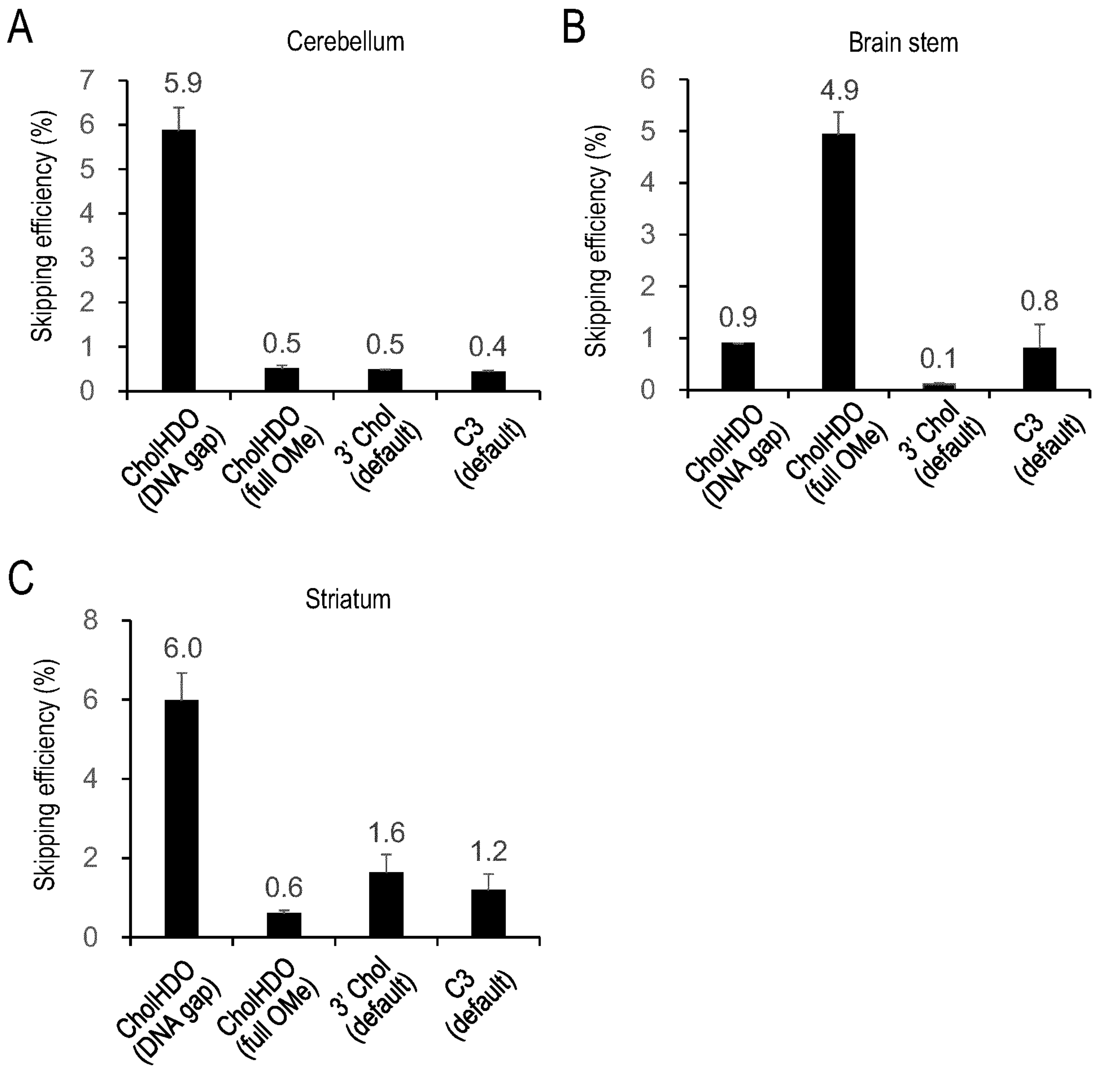
A
Cerebellum
Skipping efficiency (%)
7
6
5
4
3
2
1
0
5.9
0.5
0.5
0.4
CholHDO (DNA gap)
CholHDO (full OMe)
3' Chol (default)
C3 (default)
B
Brain stem
Skipping efficiency (%)
6
5
4
3
2
1
0
0.9
4.9
0.1
0.8
CholHDO (DNA gap)
CholHDO (full OMe)
3' Chol (default)
C3 (default)
C
Striatum
Skipping efficiency (%)
8
6
4
2
0
6.0
0.6
1.6
1.2
CholHDO (DNA gap)
CholHDO (full OMe)
3' Chol (default)
C3 (default)

Fig. 13

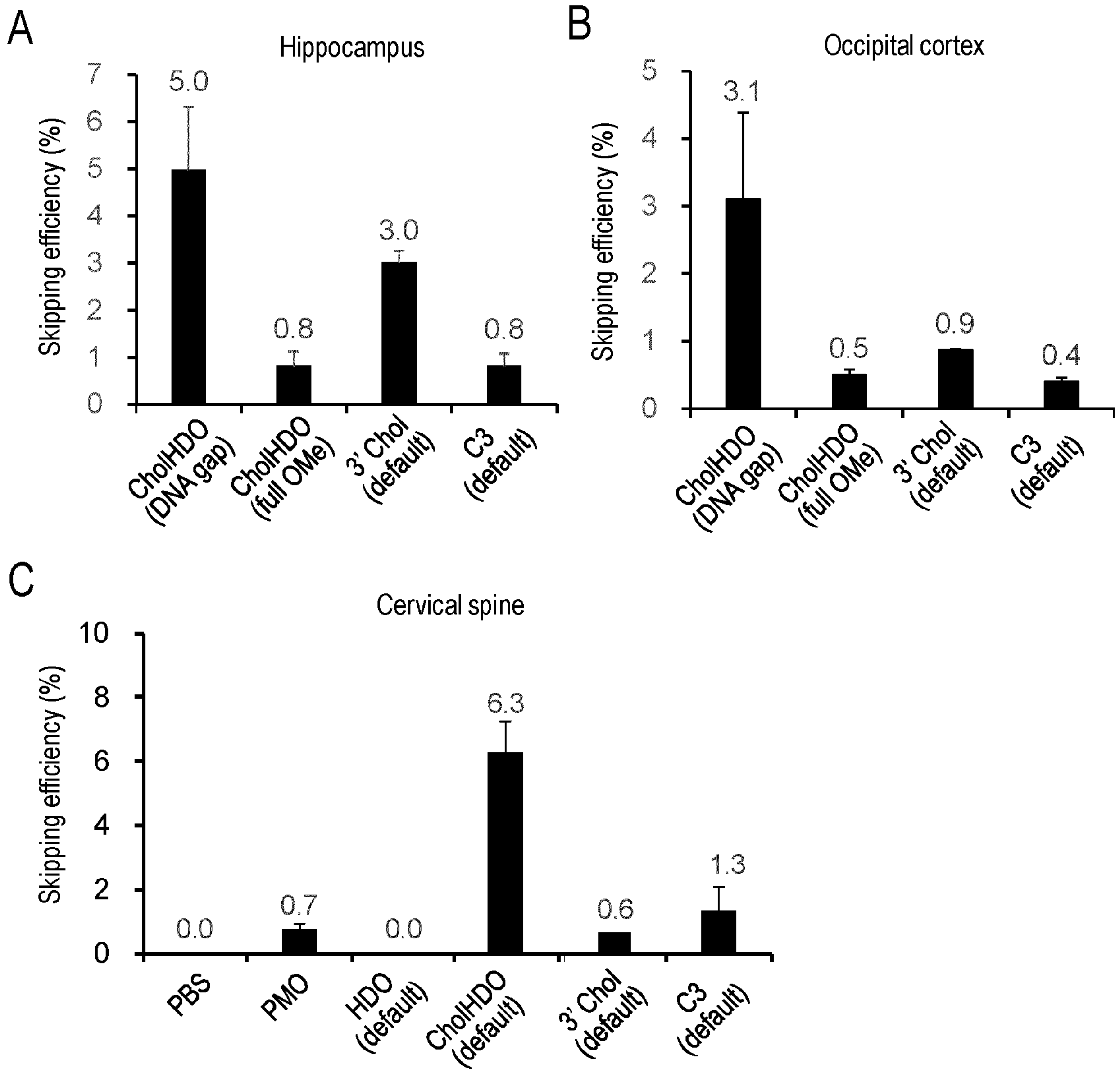
A
Hippocampus
Skipping efficiency (%)
7
6
5
4
3
2
1
0
5.0
0.8
3.0
0.8
CholHDO (DNA gap)
CholHDO (full OMe)
3' Chol (default)
C3 (default)
B
Occipital cortex
Skipping efficiency (%)
5
4
3
2
1
0
3.1
0.5
0.9
0.4
CholHDO (DNA gap)
CholHDO (full OMe)
3' Chol (default)
C3 (default)
C
Cervical spine
Skipping efficiency (%)
10
8
6
4
2
0
0.0
0.7
0.0
6.3
0.6
1.3
PBS
PMO
HDO (default)
CholHDO (default)
3' Chol (default)
C3 (default)

**INTERNATIONAL SEARCH REPORT**

International application No.
PCT/JP2021/010214

A. CLASSIFICATION OF SUBJECT MATTER
C12N 15/113(2010.01)i; A61K 31/713(2006.01)i; A61P 21/04(2006.01)i; A61P 43/00(2006.01)i; C12N 15/115(2010.01)i
FI: C12N15/113 Z ZNA; A61K31/713; A61P21/04; A61P43/00 105; C12N15/115 Z
According to International Patent Classification (IPC) or to both national classification and IPC

B. FIELDS SEARCHED

Minimum documentation searched (classification system followed by classification symbols)
C12N15/113; A61K31/713; A61P21/04; A61P43/00; C12N15/115

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | JP 2016-526529 A (NATIONAL UNIVERSITY CORPORATION TOKYO MEDICAL AND DENTAL UNIVERSITY) 05 September 2016 (2016-09-05) abstract, claims 1-19, paragraphs [0015]-[0018], [0059], [0064], [0075], [0081], [0105] | 1-14 |
| Y | JP 2015-502134 A (NATIONAL UNIVERSITY CORPORATION TOKYO MEDICAL AND DENTAL UNIVERSITY) 22 January 2015 (2015-01-22) abstract, paragraphs [0063]-[0068], [0072], [0174], [0175] | 1-14 |
| Y | JP 2019-523754 A (SAREPTA THERAPEUTICS, INC.) 29 August 2019 (2019-08-29) abstract, paragraphs [0013], [0137]-[0139] | 1-14 |
| A | JP 2016-524588 A (NATIONAL UNIVERSITY CORPORATION TOKYO MEDICAL AND DENTAL UNIVERSITY) 18 August 2016 (2016-08-18) claims 1-14, paragraphs [0069]-[0077] | 1-14 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 May 2021 (20.05.2021) | 01 June 2021 (01.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/010214

| Patent Documents referred in the Report | Publication Date | Patent Family | Publication Date |
|---|---|---|---|
| JP 2016-526529 A | 05 Sep. 2016 | WO 2014/203518 A1 abstract, claims 1-19, paragraphs [0015]-[0018], [0058], [0074], [0080], [0103]<br>US 2016/0130583 A1<br>EP 3010514 A1 | |
| JP 2015-502134 A | 22 Jan. 2015 | WO 2013/089283 A1 page 54, line 13 to page 55, line 21, page 58, lines 10-22, page 102, line 24 to page 104<br>US 2014/0302603 A1<br>US 2018/0073024 A1<br>US 2018/0320181 A1<br>US 2019/0270996 A1<br>EP 2791335 A1<br>JP 2017-140031 A<br>JP 2018-203779 A<br>JP 2020-39364 A | |
| JP 2019-523754 A | 29 Aug. 2019 | WO 2017/205513 A1 abstract, page 4, lines 24-29, page 96, line 6 to page 97, line 12<br>US 2019/0276480 A1<br>EP 3464306 A1 | |
| JP 2016-524588 A | 18 Aug. 2016 | WO 2014/192310 A1 claims 1-14, paragraphs [0057]-[0061]<br>US 2016/0145614 A1<br>EP 3004347 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013089283 A
- WO 2014192310 A
- JP 2020045137 A
- WO 2007143315 A
- WO 2008043753 A
- WO 2008049085 A
- WO 2018062510 A
- JP 2019012077 W
- JP 2019010392 W


### Non-patent literature cited in the description


- **NISHINA K.** DNA/RNA heteroduplex oligonucleotide for highly efficient gene silencing. *Nature Communication,* 2015, vol. 6, 7969
- **ASAMI Y et al.** Drug delivery system of therapeutic oligonucleotides. *Drug Discoveries & Therapeutics,* 2016, vol. 10 (5), 256-262
- **NAOKI IWAMOTO et al.** *Angew. Chem. Int. Ed. Engl.,* 2009, vol. 48 (3), 496-9
- **NATSUHISA OKA et al.** *J. Am. Chem. Soc.,* 2003, vol. 125, 8307-8317
- **NATSUHISA OKA et al.** *J. Am. Chem. Soc.,* 2008, vol. 130, 16031-16037
- **YOHEI NUKAGA et al.** *J. Org. Chem.,* 2016, vol. 81, 2753-2762
- **YOHEI NUKAGA et al.** *J. Org. Chem.,* 2012, vol. 77, 7913-7922
- **NAOKI IWAMOTO et al.** *Nat. Biotechnol,* 2017, vol. 35 (9), 845-851
- **ANASTASIA KHVOROVA et al.** *Nat. Biotechnol.,* 2017, vol. 35 (3), 238-248
- **NISHINA K.** *Nature Communication,* 2015, vol. 6, 7969
- **ASAMI Y et al.** *Drug Discoveries & Therapeutics,* 2016, vol. 10 (5), 256-262